# EUROPÄISCHE PATENTANMELDUNG

(11) **EP 4 124 331 A1**
(43) Veröffentlichungstag der Anmeldung: **01.02.2023**
(21) Anmeldenummer: 21188081.0
(22) Anmeldetag: 27.07.2021
(51) Int. Cl.: A61K 6/16, A61K 6/17, A61K 6/62, A61K 6/65, A61K 6/71, A61K 6/76, A61K 6/77, A61K 6/78, A61K 6/818, A61K 6/887

(54) **ÄSTHETISCHES DENTALES FÜLLUNGSMATERIAL MIT HOHER DURCHHÄRTUNGSTIEFE**

(71) Anmelder: Ivoclar Vivadent AG, 9494 Schaan (LI)
(72) Erfinder: BIELEC, Philipp, 88142 Wasserburg (Bodensee) (DE); GEBHARDT, Benjamin, 9472 Grabs (CH)
(74) Vertreter: Uexküll & Stolberg

(57) **Zusammenfassung**

Dentalwerkstoff, der (a) mindestens ein radikalisch polymerisierbares Monomer, (b) mindestens einen röntgenopaken Füllstoff, (c) mindestens einen anorganischen Füllstoff, (d) mindestens einen Kompositfüllstoff und (e) mindestens einen Initiator für die radikalische Polymerisation enthält, wobei das Monomer (a) mindestens ein Urethan(meth)acrylat, mindestens ein radikalisch polymerisierbaren Bisphenol A-Derivat, ggf. mindestens ein polycyclischaliphatisches Dimethacrylat, ggf. mindestens ein Monomer, das nicht in eine der Gruppen (a-1) bis (a-3) und (a-5) fällt, und ggf. mindestens einen Kettenregler (a-5) enthält. Der Dentalwerkstoff erlaubt die Herstellung ästhetisch ansprechender Restaurationen mit nur einem Material.

## Beschreibung

### Ästhetisches dentales Füllungsmaterial mit hoher Durchhärtungstiefe

Die vorliegende Erfindung betrifft röntgenopake Dentalwerkstoffe, die sich durch eine große Durchhärtungstiefe auszeichnen und eine vereinfachte Herstellung ästhetisch ansprechender Dentalrestaurationen erlauben. Die Werkstoffe eignen sich insbesondere als dentale Füllungsmaterialien.

### Stand der Technik

Dentale Füllungsmaterialien auf Methacrylatbasis werden oft als Kunststofffüllungen oder korrekter als Komposite bezeichnet. Kompositmaterialien enthalten eine polymerisierbare organische Matrix und Füllstoffe sowie diverse Zuschlagsstoffe, wie Stabilisatoren, Initiatoren und Pigmente. Der Füllstoffgehalt hängt maßgeblich vom gewünschten Verwendungszweck ab und kann bis zu 90 Gew.-% betragen.

Die polymerisierbare organische Matrix von dentalen Füllungskompositen und Adhäsiven basiert meist auf einer Mischung von Dimethacrylaten, die häufig das hochviskose Bis-GMA als Vernetzer enthalten. Bis-GMA führt zu guten mechanischen Eigenschaften bei vergleichsweise geringem Volumenschrumpf. Weitere Beispiele für häufig eingesetzte Dimethacrylate sind Urethandimethacrylate und die regelmäßig als Verdünnermonomere genutzten, niedrigviskosen Dimethacrylate Bis(methacryloyloxymethyl)tricyclo[5.2.1.]decan (TCDMA), Decandiol-1,10-dimethacrylat (D₃MA) und Triethylenglycoldimethacrylat (TEGDMA).

Die polymerisierbare organische Matrix enthält in der Regel einen Initiator für die radikalische Polymerisation, wobei lichthärtende Werkstoffe, die einen Photoinitiator enthalten, heutzutage in der zahnärztlichen Füllungstherapie eine dominante Stellung einnehmen. Ein Nachteil von lichthärtenden Materialien ist, dass besonders das Legen großer Füllungen aufwändig ist, weil das zur Härtung erforderliche Licht nur bis zu einer begrenzten Tiefe in die Materialien eindringen kann. Bei der sogenannten Inkrementtechnik wird die Füllung daher schichtweise aus dem Kompositwerkstoff aufgebaut, wobei die Schichten eine Dicke von jeweils ca. 2 mm haben und einzeln gehärtet werden müssen.

Diesen Nachteil überwinden sogenannte Bulk-Fill-Materialien, die Durchhärtungstiefen von ca. 4 mm pro Schicht erlauben. Übliche Füllstoffe weisen eine Durchhärtungstiefe von etwa 2 mm auf. Bulk-Fill-Materialien verfügen jedoch oft nicht über die gewünschten ästhetischen Eigenschaften und sind daher nicht oder nur eingeschränkt für die Frontzahnrestauration geeignet. Die Durchhärtungstiefe korreliert mit der Transluzenz der Werkstoffe, wobei eine hohe Transluzenz und eine gute Durchhärtungstiefe dann erreicht werden, wenn die organische Matrix und die verwendeten Füllstoffe übereinstimmende Brechungsindices aufweisen. Nachteilig hierbei ist, dass solche Komposite aufgrund ihrer hohen Transluzenz das darunterliegende Dentin nur schlecht abdecken, was aus ästhetischen Gründen störend ist, weil die Farbe des Dentins von der des sichtbaren Zahnschmelzes abweicht.

Die WO 2016/026915 A1 offenbart radikalisch polymerisierbare Dentalwerkstoffe, die eine hohe Durchhärtungstiefe mit guter Opazität kombiniert. Die Werkstoffe sind dadurch gekennzeichnet, dass die zu ihrer Herstellung verwendete Monomermischung einen Brechungsindex n_{D} von 1,50 bis 1,70 aufweist und dass der Brechungsindex der Monomermischung vor der Härtung dem Brechungsindex des Füllstoffs entspricht oder um maximal 0,013 größer ist, nach der Härtung aber um mindestens 0,02 größer als der Brechungsindex des Füllstoffs ist. Die Dentalwerkstoffe weisen vor der Polymerisation eine hohe Transluzenz und damit eine große Durchhärtungstiefe auf. Bei der Polymerisation nimmt die Transluzenz ab. Die Materialien können röntgenopake Füllstoffe wie z.B. röntgenopake Gläser oder Ytterbiumfluorid mit einer Partikelgröße von 0,050 bis 2,0 µm enthalten. Die Materialien sind als Bulk-Fill-Materialien geeignet, sind aber aufgrund ihrer Fließfähigkeit nicht stopfbar.

Die US 10,828,240 offenbart radikalisch polymerisierbare Dentalwerkstoffe, die radikalisch polymerisierbares Monomer, anorganischen Füllstoff und organisch-anorganischen Füllstoff enthalten und die eine hohe Durchhärtungstiefe aufweisen sollen. Dies soll dadurch erreicht werden, dass die Brechungsindizes der Füllstoffe im Bereich des Brechungsindexes des Monomers liegt, wobei In den Ausführungsbeispielen der Brechungsindex der Monomere kleiner als der Brechungsindex der Füllstoffe ist.

EP 2 965 741 A1 offenbart radikalisch polymerisierbare Dentalwerkstoffe, die bei guten mechanischen Eigenschaften einen verzögerten Gelpunkt und eine verringerte Polymerisationsschrumpfungsspannung in Kombination mit einer homogenen Netzwerkarchitektur, einer engen und niedrigen Glasübergangstemperatur und verbesserte Schlagzähigkeit aufweisen. Diese Verbesserungen werden durch die Verwendung spezieller Kettenübertragungsreagenzien (Kettenregler) erreicht.

Die US 4,629,746 offenbart mikrogefüllte Dentalwerkstoffe, die Seltenerdmetallfluoride wie Ytterbiumfluorid mit einer Primärteilchengröße von 5 bis 700 nm, vorzugsweise 50 bis 300 nm als röntgenopake Füllstoffe enthalten. Neben den röntgenopaken Füllstoffen können die Materialien nicht röntgenopake Füller wie gefällte oder pyrogene Kieselsäuren enthalten. Die Werkstoffe sollen eine hohe Röntgenopazität und eine gute Transmission aufweisen.

Die EP 1 234 567 A2 offenbart Präpolymerisate mit definierter Korngrößenverteilung, die nur einen geringen Anteil feinkörniger Partikel mit einer Größe von weniger als 10 µm enthalten. Diese Füllstoffe sollen polymerisierbare Zusammensetzungen mit geringem Polymerisationsschrumpf und guter Polierbarkeit, Oberflächenglätte und Abrasionsbeständigkeit ergeben. Zur Erhöhung der Röntgenopazität können die Präpolymerisate röntgenopake Füllstoffe wie Ytterbiumfluorid mit einer Partikelgröße von 300 nm enthalten.

Die WO 2017/149242 A1 offenbart die Herstellung von kolloidalen Suspensionen von Ytterbiumfluorid mit einer Partikelgröße von weniger als 100 nm und deren Verwendung zur Herstellung von Dentalwerkstoffen.

Die US 9,833,388 B2 offenbart Dentalwerkstoffe, die Ytterbiumfluorid mit einer Partikelgröße zwischen 25 und 120 nm enthalten. Diese sollen sich durch eine geringe Zahl von Artefakten bei der Volumentomographie auszeichnen.

Die EP 2 965 741 A1 offenbart die Verwendung von radikalisch polymerisierbaren schwefelhaltigen Monomeren wie 2-(Toluol-4-sulfonylmethyl)acrylsäurelaurylester als Kettenregler zur Verringerung der Polymerisationsschrumpfungsspannung (PKS) in Dentalwerkstoffen.

### Aufgabenstellung

Gemäß ISO4049:2019 ist ein röntgenopaker Dentalwerkstoff als ein Material definiert, das mindestens die gleiche Röntgenopazität besitzt, wie das Element Aluminium. Um in einem Röntgenbild gut gesehen werden zu können, ist es empfehlenswert, dass Dentalmaterialien eine doppelt so hohe Röntgenopazität (200 % AI) wie Aluminium aufweisen, da die Röntgenopazität von natürlichem Zahnschmelz ca. 170 % AI entspricht. Um Kompositmaterialien die gewünschte Röntgenopazität zu verleihen, werden röntgenopake Füllstoffe zugesetzt. Hierbei handelt es sich meist um barium- oder strontiumhaltige Gläser, die einen relativ hohen Brechungsindex aufweisen.

Die Durchhärtungstiefe von Dentalwerkstoffen korreliert positiv mit der Transluzenz der Werkstoffe. Um Dentalmaterialien in einer Schichtdicke von 4 mm in einem Belichtungsschritt polymerisieren zu können, ist eine hohe Transluzenz erforderlich, wobei eine hohe Transluzenz und damit ein hohe Durchhärtungstiefe dann erreicht werden, wenn der Brechungsindex der organischen Matrix mit den Brechungsindices der verwendeten Füllstoffe übereinstimmt. Eine hohe Transluzenz hat den zusätzlichen Vorteil, dass sich die Füllung farblich gut an den Zahn anpasst. Nachteilig daran ist jedoch, dass eine hohe Transparenz eine geringe Opazität zur Folge hat, die sich von der des natürlichen Zahns unterscheidet. Im Frontzahn sorgt das für unnatürlich grau/dunkel wirkende Füllungen, da die dunkle Mundhöhle durchscheint, während im Seitenzahn das durchscheinende Dentin optisch nachteilig ist. Ein weiteres Problem bei der Herstellung von röntgenopaken Dentalwerkstoffen mit hoher Durchhärtungstiefe ist der hohe Brechungsindex der Glasfüllstoffe. Dieser schränkt die Auswahl an geeigneten Monomeren sehr ein.

Herkömmliche Dentalwerkstoffe sind in der Regel für bestimmte Anwendungen optimiert, so dass eine Vielzahl unterschiedlicher Materialien verfügbar ist. Zur Erzielung eines medizinisch wie optisch befriedigenden Behandlungserfolgs ist es meist erforderlich, unterschiedliche Materialien auf geeignete Weise miteinander zu kombinieren. Dies macht die Behandlung zeitaufwändig und teuer.

Der Erfindung liegt, die Aufgabe zugrunde, Dentalwerkstoffe zur Verfügung zu stellen, welche die zuvor genannten Nachteile nicht aufweisen. Die Dentalwerkstoffe sollen die Herstellung ästhetisch ansprechender und medizinisch in jeder Hinsicht befriedigender Restaurationen mit nur einem Material ermöglichen. Die Dentalwerkstoffe sollen Restaurationen ergeben, die hinsichtlich Opazität, Transluzenz und Farbe gut mit der natürlichen Zahnhartsubstanz zusammenpassen. Darüber sollen die Materialien eine hohe Glanzstabilität und gute Polierbarkeit sowie eine geringe Oberflächenrauheit aufweisen und röntgenopak sein.

Diese Aufgabe wird erfindungsgemäß durch Dentalwerkstoffe gelöst, die
(a) mindestens ein radikalisch polymerisierbares Monomer,
(b) mindestens einen röntgenopaken Füllstoff,
(c) mindestens einen anorganischen Füllstoff,
(d) mindestens einen Kompositfüllstoff und
(e) mindestens einen Initiator für die radikalische Polymerisation enthalten.

Es wurde gefunden, dass durch eine gezielte Auswahl an sich bekannter Stoffe Dentalwerkstoffe hergestellt werden können, die die obigen Anforderungen erfüllen.

### Monomer (a)

Als **Monomer (a)** sind radikalisch polymerisierbare, polyfunktionelle Monomere und insbesondere (Meth)acrylamide und (Meth)acrylate bevorzugt. Besonders bevorzugt sind monofunktionelle und polyfunktionelle Methacrylate, ganz besonders bevorzugt monofunktionelle und difunktionelle Methacrylate sowie Mischungen davon. Weiter bevorzugt sind difunktionelle Hybridmonomere. Hybridmonomere sind Monomere, die sowohl (Meth)acrylamid- als auch (Meth)acrylatgruppen enthalten. Unter polyfunktionellen Monomeren werden Verbindungen mit zwei oder mehr, vorzugsweise 2 bis 4 und insbesondere 2 radikalisch polymerisierbaren Gruppen verstanden.

Das Monomer (a) kann ein einzelnes Monomer oder vorzugsweise eine Mischung von unterschiedlichen Monomeren enthalten. Erfindungsgemäß besonders bevorzugt sind Dentalwerkstoffe, in denen die Komponente (a) eine Mischung der folgenden Monomere enthält:
(a-1) 20 bis 80 Gew.-%, bevorzugt 25 bis 55 Gew.-% und ganz besonders bevorzugt 30 bis 50 Gew.-% mindestens eines Urethan(meth)acrylats,
(a-2) 8 bis 45 Gew.-%, besonders bevorzugt von 10 bis 35 Gew.-% und ganz besonders bevorzugt 12 bis 35 Gew.-% mindestens eines radikalisch polymerisierbaren Bisphenol A-Derivats,
(a-3) 10 bis 50 Gew.-%, besonders bevorzugt von 15 bis 40 Gew.-% und ganz besonders bevorzugt 20 bis 35 Gew.%, mindestens eines polycyclisch-aliphatischen, vorzugsweise tricyclisch-aliphatischen Dimethacrylats, besonders bevorzugt Tricyclodecandimethanoldimethacrylat (TCP),
(a-4) 0 bis zu 20 Gew.-%, besonders bevorzugt 2 bis 20 Gew.-% und ganz besonders bevorzugt von 4 bis 10 Gew.-% sonstige Monomere, d.h. Monomere, die nicht in eine der Gruppen (a-1) bis (a-3) und (a-5) fallen,
(a-5) 0 bis zu 8 Gew.-%, vorzugsweise 0,5 bis 7 Gew.-% und besonders bevorzugt 1 bis 6 Gew.-% mindestens eines Kettenreglers,
jeweils bezogen auf die Gesamtmasse der Komponente (a).

Die Monomere (a-1) bis (a-5) werden vorzugsweise jeweils aus den folgenden Stoffen ausgewählt, wobei erfindungsgemäß solche Dentalwerkstoffe besonders bevorzugt sind, in denen die Komponente (a) ausschließlich die genannten Monomere enthält. In allen Fällen können einzelne Monomere oder eine Mischung von mehreren Monomeren eingesetzt werden. Der Begriff (Meth)acrylat steht für Acrylat, Methacrylat oder eine Mischung davon, wobei in allen Fällen die Bedeutung Methacrylat bevorzugt ist.

Bevorzugte **Urethan(meth)acrylate (a-1)** sind Urethandi(meth)acrylate und insbesondere Urethandimethacrylate.

Besonders bevorzugt sind Urethandi(meth)acrylate mit aromatischen Gruppen, insbesondere die in EP 0 934 926 A1 beschriebenen Urethandi(meth)acrylatderivate von 1,3-Bis(1-iso-cyoanato-1-methylethyl)benzol, wobei Tetramethyl-xylylen-diurethan-di(meth)acrylat (V380) ganz besonders bevorzugt ist:

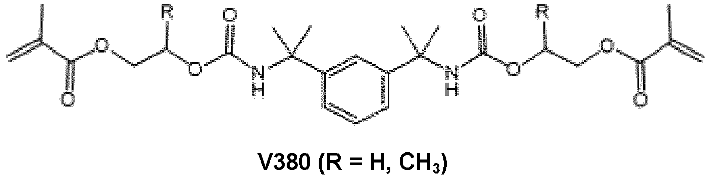

In der gezeigten Formel sind die Reste R unabhängig voneinander H oder CH₃, wobei die Reste die gleiche Bedeutung oder unterschiedliche Bedeutungen haben können. Vorzugsweise wird eine Mischung eingesetzt, die Moleküle, in denen beide Reste H sind, Moleküle, in denen beide Reste CH₃ sind, und Moleküle enthält, in denen ein Rest H und der andere Rest CH₃ ist (CAS-Nummern: 17884-94-1 (R = H), 138393-21-2 (R = CH₃), 1219495-43-8 (R = H, CH₃)). Eine solche Mischung ist beispielsweise durch Reaktion von 1,3-Bis(1-isocyana-to-1-methylethyl)benzol mit Hydroxypropylmethacrylat und 2-Hydroxyethylmethacrylat erhältlich. Ganz besonders bevorzugt ist Tetramethyl-xylylen-diurethan-dimethacrylat (R = CH₃).

Urethandimethacrylatmonomere mit aromatischen Gruppen werden vorzugsweise in einer Gesamtmenge von 5 bis 60 Gew.-%, besonders bevorzugt von 10 bis 45 Gew.-% und ganz besonders bevorzugt 10 bis 25 Gew.-% bezogen auf die Masse der Monomerkomponente (a) eingesetzt.

Weiter bevorzugt sind Urethandi(meth)acrylate der allgemeinen Formel I: 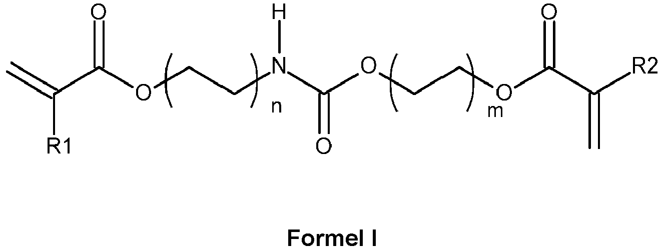 in der die Variablen die folgenden Bedeutungen haben:
- R¹, R² =: unabhängig voneinander jeweils C₁-C₈-Alkyl, vorzugsweise C₁-C₄-Alkyl, besonders bevorzugt C₁-C₂-Alkyl, ganz besonders bevorzugt CH₃; wobei R¹ und R² vorzugsweise gleich sind,
- n, m =: unabhängig voneinander jeweils eine ganze Zahl von 1 bis 2 und bevorzugt 1.

Ein besonders bevorzugtes Urethandimethacrylat der Formel 1 ist N-(2-Methacryloyl-oxyethyl)carbaminsäure-(2-methacryloyloxyethyl)ester (V837; CAS-Nr. 139096-43-8):

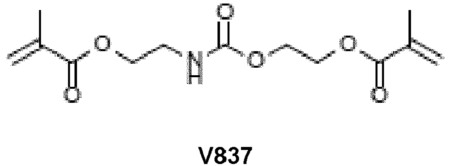

Wenn vorhanden, werden die Urethandi(meth)acrylate der Formel I in einer Menge von 5 Gew.-% oder weniger, vorzugsweise in einer Menge von 0,01 bis 5 Gew.-%, besonders bevorzugt 0,04 bis 4 Gew.-%, bezogen auf die Masse der Monomerkomponente (a), eingesetzt.

Neben den genannten Urethandi(meth)acrylaten können die erfindungsgemäßen Dentalwerkstoffe vorteilhaft weitere Urethandi(meth)acrylate enthalten, vorzugsweise Urethandimethacrylate. Diese werden vorzugsweise in einer Menge von 10 bis 70 Gew.-%, besonders bevorzugt 15 bis 45 Gew.-% und ganz besonders bevorzugt 15 bis 35 Gew.-% bezogen auf die Masse der Monomerkomponente (a) eingesetzt. Ein besonders bevorzugtes Urethandimethacrylat ist 7,7(9)9-Trimethyl-4,3-dioxo-3,14-dioxa-5,12-diazohexadecan-1,16-diyl-dimethacrylat (RM3; ein Additionsprodukt aus 2-Hydroxyethylmethacrylat und 2,2,4-Trimethylhexamethylendiisocyanat).

Als Komponente (a-1) wird vorzugsweise eine Monomermischung eingesetzt, die
- 5 bis 60 Gew.-%, bevorzugt von 10 bis 45 Gew.-% und besonders bevorzugt 10 bis 25 Gew.-% mindestens eines Urethandimethacrylatmonomers mit aromatischen Gruppen, vorzugsweise V380,
- 0 bis zu 5 Gew.-%, vorzugsweise 0,01 bis zu 5 Gew.-% und besonders bevorzugt 0,04 bis 4 Gew.-% mindestens eines Urethandi(meth)acrylats der Formel I, vorzugsweise V837 und
- 10 bis 70 Gew.-%, besonders bevorzugt 15 bis 45 Gew.-% und ganz besonders bevorzugt 15 bis 35 Gew.-% mindestens eines weiteren Urethandimethacrylats, vorzugsweise RM3, enthält,
jeweils bezogen auf die Gesamtmasse der Monomerkomponente (a).

Die Gesamtmenge an Urethan(meth)acrylaten (a-1) liegt vorzugsweise im Bereich von 20 bis 80 Gew.-%, vorzugsweise 25 bis 55 Gew-% und besonders bevorzugt 30 bis 50 Gew.-%, bezogen auf die Masse der Monomerkomponente (a).

Bevorzugte **radikalisch polymerisierbare Bisphenol A-Derivate (a-2)** sind Bisphenol A-Dimethacrylate, insbesondere 2,2-Bis[4-(2-hydroxy-3-methacryloyloxypropyl)phenyl]propan (Bis-GMA) oder 2,2-Bis(4-methacryloyloxyphenyl)propane (Bisphenol A-Dimethacrylat; (CAS-Nr. 3253-39-2), besonders bevorzugt ethoxyliertes oder propoxyliertes Bisphenol-A-Dimethacrylat und ganz besonders bevorzugt 2-[4-(2-Methacryloyloxyethoxyethoxy)phenyl]-2-[4-(2-methacryloyloxyethoxy)phenyl]propan) (SR-348c, enthält 3 Ethoxygruppen).

Das oder die Bisphenol A-Derivate (a-2) werden vorzugsweise in einer Gesamtenge von 8 bis 45 Gew.-%, besonders bevorzugt von 10 bis 35 Gew.-% und ganz besonders bevorzugt 12 bis 35 Gew.-% eingesetzt, bezogen auf die Masse der Monomerkomponente (a).

Bevorzugte **polycyclisch-aliphatische Dimethacrylate (a-3)** sind, cyclische, nichtaromatische Dimethacrylate, wobei der polycyclisch-aliphatische Rest vorzugsweise 5 bis 20 Kohlenstoffatome enthält und optional ein Heteroatom enthalten kann, besonders bevorzugt 5 bis 15 Kohlenstoffatomen enthält und optional ein Sauerstoff-, Stickstoff- oder Schwefelatom enthalten kann.

Ganz besonders bevorzugte polycyclisch-aliphatische Dimethacrylate (a-3) sind tricyclische Dimethacrylate wie Tricyclodecandimethanoldimethacrylate und ganz besonders bevorzugt Tricyclodecandimethanoldimethacrylat TCP (CAS-Nummer: 42594-17-2). TCP verändert seinen Brechungsindex bei der Polymerisation von 1,501 nach 1,531.

Polycyclisch-aliphatische Dimethacrylate werden vorzugsweise in einer Gesamtmenge von 10 bis 50 Gew.-%, besonders bevorzugt von 15 bis 40 Gew.-% und ganz besonders bevorzugt 20 bis 35 Gew.%, bezogen auf die Masse der Monomerkomponente (a), eingesetzt.

Als **weitere Monomere (a-4)** sind (Meth)acrylamide, z.B. N-disubstituierte (Meth)acrylamide, wie N,N-Dimethylacrylamid, sowie Bis(meth)acrylamide, wie N,N'-Diethyl-1,3-bis(acryl-amido)propan, 1,3-Bis(methacrylamido)-propan, 1,4-Bis(acrylamido)-butan und 1,4-Bis(acryloyl)piperazin bevorzugt. Besonders bevorzugt sind monofunktionelle Methacrylate sowie polyfunktionelle, vorzugsweise difunktionelle, Methacrylate, wie Di-, Tri- oder Tetraethylenglycoldimethacrylat, Trimethylolpropantrimethacrylat, Pentaerythrittetramethacrylat, Glycerindimethacrylat und Glycerintrimethacrylat, 1,4-Butandioldimethacrylat, 1,10-Decandioldimethacrylat (D₃MA), 1,12-Dodecandioldimethacrylat. Gemäß einer besonders bevorzugten Ausführungsform enthalten die erfindungsgemäßen Dentalwerkstoffe mindestens ein monofunktionelles Methacrylat, besonders bevorzugt 2-([1,1'-Biphenyl]-2-oxy)ethylmethacrylat (MA836), oder eine Mischung von mono- und difunktionellen Methacrylaten, insbesondere eine Mischung von 2-([1,1'-Biphenyl]-2-oxy)ethylmethacrylat (MA836) und 1,10-Decandioldimethacrylat (D₃MA).

1,10-Decandioldimethacrylat (D₃MA) und 2-([1,1'-Biphenyl]-2-oxy)ethylmethacrylat (MA-836) zeichnen sich durch einen großen Brechungsindexunterschied zwischen Monomer- und Polymerform aus (1,460 zu 1,500 für D₃MA und 1,575 zu 1,598 MA-836). D₃MA hat einen sehr niedrigen und MA-836 einen sehr hohen Brechungsindex. Die Monomere eignen sich daher besonders zur Einstellung des Brechungsindexes der Monomerkomponente (a).

Die erfindungsgemäßen Zusammensetzungen können als Monomer (a-4) weiterhin ein oder mehrere sog. Hybridmonomere enthalten. Hierunter werden Monomere verstanden, die mindestens eine (Meth)acrylamid- und eine (Meth)acrylatgruppe enthalten. Bevorzugte Monomere dieses Typs sind die in EP 3 064 192 A1 offenbarten Hybridmonomere, wobei Monomere mit Methacrylamid- und Methacrylatgruppen besonders bevorzugt sind. Ganz besonders bevorzugt sind Hybridmonomere, die zusätzlich eine Urethangruppe aufweisen.

Monomere (a-4) werden vorzugsweise in einer Gesamtmenge von maximal 20 Gew.-%, besonders bevorzugt 2 bis 20 Gew.-% und ganz besonders bevorzugt von 4 bis 10 Gew.-% eingesetzt, bezogen auf die Masse der Monomerkomponente (a). Die Gesamtmenge an monofunktionellen radikalisch polymerisierbaren Monomeren beträgt hierbei vorzugsweise 20 Gew.-% oder weniger, besonders bevorzugt 1 bis 17 Gew.-%, ganz besonders bevorzugt 2 bis 14 Gew.-% und am meisten bevorzugt 3 bis 11 Gew.-% bezogen auf die Monomerkomponente (a). Monofunktionelle radikalisch polymerisierbare Monomeren sind nach der Polymerisation leichter aus dem gehärteten Werkstoff auswaschbar als polyfunktionelle Monomere, was unter toxikologischen Gesichtspunkten unerwünscht ist. Aus diesem Grund sind erfindungsgemäß Dentalwerkstoffe bevorzugt, die bezogen auf die Gesamtmasse des Dentalwerkstoffs weniger als 2 Gew.-%, besonders bevorzugt 0,1 bis 1,9 Gew.-%, ganz besonders bevorzugt 0,2 bis 1,8 Gew.-% und am meisten bevorzugt 1,3 bis 1,7 Gew.-% an monofunktionellen radikalisch polymerisierbaren Monomeren enthalten.

Bevorzugte **Kettenregler (a-5)** sind die in der EP 2 965 741 A1 offenbarten, radikalisch polymerisierbaren, schwefelhaltigen Monomere. Besonders bevorzugt werden als Kettenregler (a-5) Verbindungen aus der Gruppe der Allylsulfone verwendet. Besonders geeignete Allylsulfone sind 2-(Toluol-4-sulfonylmethyl)-acrylsäureethylester (TSMEA), 2-(Toluol-4-sulfonylmethyl)-acrylsäure-2-(2-ethoxy-ethoxy)-ethylester, 2-(Toluol-4-sulfonylmethyl)-acrylsäure, 2-(Toluol-4-sulfonylmethyl)-acrylsäuretert-butylester, 3-(Trimethoxysilyl)propyl-2-(to-sylmethyl)acrylat, 3-(Triethoxysilyl)propyl-2-(tosylmethyl)acrylat, 3-(Triethoxysilyl)propyl-2-(tosylmethyl)acrylamid, 2-(Methylsulfonyl)-ethylmethacrylat, 2-(Methylsulfonyl)-ethylmethacrylat, 3-(Methylsulfonyl)-propylmethacrylat, Triethylenglycol-bis[2-(toluol-4-sulfonylmethyl)-acrylat], 2-(Toluol-4-sulfonylmethyl)-acrylsäure-(2-methacryloyloxyethyl)ester, 2-(Toluol-4-sulfonylmethyl)-acrylsäure-(6-methacryloyloxyhexyl)ester, 2-(Toluol-4-sulfonylmethyl)acrylsäure-(10-methacryloyloxydecyl)ester, 2-(Toluol-4-sulfonylmethyl)-acrylsäure-(2-hydroxy-3-methacryloyloxypropyl)ester, 2-(Toluol-4-sulfonylmethyl)-acrylsäure-(8-methacryloyloxy-3,6-dioxaoctyl)ester, wobei 2-(Toluol-4-sulfonylmethyl)-acrylsäureethylester (TSMEA) ganz besonders geeignet ist. Kettenregler sind Monomere, die das Kettenwachstum während der Polymerisation steuern. Hierdurch wird eine Verringerung der Schrumpfkraft erreicht. Eine geringe Schrumpfkraft wirkt sich vorteilhaft auf die Randdichtigkeit von Füllungen aus. Kettenregler werden auch als Kettenüberträger bezeichnet.

Kettenregler werden vorzugsweise in einer Menge von 0 bis 8 Gew.-%, besonders bevorzugt von 0,5 bis 7 Gew.-% und ganz besonders bevorzugt von 1 bis 6 Gew.-%, bezogen auf die Masse der Monomerkomponente (a), eingesetzt.

Die Gesamtmenge an radikalisch polymerisierbaren Monomeren (a) liegt vorzugsweise in einem Bereich von 5 bis 40 Gew.-%, besonders bevorzugt von 10 bis 35 Gew.-%, ganz besonders bevorzugt von 12 bis 30 Gew.-%, und am meisten bevorzugt von 15 bis 25 Gew.-%, bezogen auf die Gesamtmasse des Dentalwerkstoffs.

Erfindungsgemäß besonders bevorzugt sind Dentalwerkstoffe, die als Komponente (a-1) Tetramethyl-xylylen-diurethan-dimethacrylat (V380), 1,6-Bis-[2-methacryloyloxy-ethoxy-carbonylamino]-2,2,4-trimethylhexan (RM3), N-(2-Methacryloyloxyethyl)carbaminsäure-(2-methacryloyloxyethyl)ester (V837; CAS-Nr. 139096-43-8) oder eine beliebige Mischung davon; als Komponente (a-2) Bisphenol-A-dimethacrylat, ethoxy- oder propoxyliertes Bisphenol-A-Dimethacrylat, insbesondere Bisphenol-A-Dimethacrylat 2-[4-(2-Methacryloyl-oxyethoxyethoxy)phenyl]-2-[4-(2-methacryloyloxyethoxy)phenyl]propan) (SR-348c), 2,2-Bis-[4-(2-hydroxy-3-methacryloyloxypropyl)phenyl]propan (Bis-GMA) oder eine beliebige Mischung davon; als Komponente (a-3) Bis-(3-methacryloyloxymethyl)tricyclo-[5.2.1.0^{2,6}]decan (TCP); als Komponente (a-4) 2-([1,1'-Biphenyl]-2-oxy)ethylmethacrylat (MA-836) und/oder 1,10-Decandioldimethacrylat (D₃MA); und als Komponente (a-5) 2-(Toluol-4-sulfonylmethyl)-acrylsäureethylester (MA-769) enthalten.

Die Monomerkomponente (a) hat vorzugsweise einen Brechungsindex von 1,495 bis 1,520, besonders bevorzugt von 1,505 bis 1,515. Der Brechungsindex der Monomermischung wird vorzugsweise so eingestellt, dass er vor der Härtung dem Brechungsindex des Füllstoffs (c) entspricht oder maximal 0,03 darüber liegt. Vorzugsweise ist der Brechungsindex des Monomers oder der Monomermischung um 0,002 bis 0,02, besonders bevorzugt um 0,005 bis 0,015 größer als der Brechungsindex des Füllstoffs (c). Der Brechungsindex der Komponente (a) kann durch das Mischen von Monomeren mit unterschiedlichen Brechungsindices eingestellt werden. Aufgrund des geringen Unterschieds der Brechungsindices von Monomer und Füllstoff weisen die erfindungsgemäßen Dentalwerkstoffe vor der Polymerisation eine hohe Transluzenz auf, so dass das zur Polymerisation eingesetzte Licht tief in die Materialien eindringen kann, was eine große Durchhärtungstiefe gewährleistet. Bei der Polymerisation nimmt der Brechungsindex der Monomere zu, während der Brechungsindex des Füllstoffs unverändert bleibt. Dadurch vergrößert sich die Differenz zwischen den Brechungsindices von Monomer und Füllstoff, und die Transluzenz nimmt dementsprechend ab. Das ist aus ästhetischen Gründen vorteilhaft, weil tiefer liegende Schichten des Zahns mit einer anderen Färbung besser abgedeckt werden.

Erfindungsgemäß sind Monomere und Monomermischungen bevorzugt, die bei der Polymerisation eine große Änderung des Brechungsindexes zeigen. Die als Komponente (a) verwendeten Monomere werden vorzugsweise so ausgewählt, dass der Brechungsindexunterschied zwischen dem unpolymerisierten und dem polymerisierten Zustand mindestens 0,015, vorzugsweise mindestens 0,02 beträgt. Gemäß einer besonders bevorzugten Ausführungsform beträgt der Brechungsindexunterschied 0,015 bis 0,04, besonders bevorzugt von 0,021 bis 0,035 und ganz besonders bevorzugt von 0,022 bis 0,030.

### Röntgenopake Füllstoffe (b)

Die erfindungsgemäßen Werkstoffe enthalten als **Komponente (b)** mindestens einen röntgenopaken Füllstoff, vorzugsweise Tantal(V)oxid, Bariumsulfat, ein Mischoxid von SiO₂ mit Ytterbium(III)oxid oder Tantal(V)oxid, ein Seltenerdfluorid, bevorzugt Ytterbiumtrifluorid, oder eine Mischung davon, wobei Ytterbiumtrifluorid (YbF₃) besonders bevorzugt ist.

Der oder die röntgenopaken Füllstoffe (b) liegen in partikulärer Form vor und haben vorzugsweise eine mittlere Teilchengröße von ≤ 25 nm, vorzugsweise < 25 nm, besonders bevorzugt von 10 bis 24 nm und ganz besonders bevorzugt von 15 bis 24 nm, wobei die Partikel in nicht aggregierter und nicht agglomerierter Form vorliegen. Partikel mit einer Teilchengröße von ≤ 25 nm werden hierin als nanoskalig bezeichnet. Ganz besonders bevorzugt enthalten die erfindungsgemäßen Werkstoffe als röntgenopaken Füllstoff (b) Ytterbiumtrifluorid-Partikel mit einer mittleren Primärteilchengröße von ≤ 25 nm, vorzugsweise < 25 nm, besonders bevorzugt von 10 bis 24 nm ganz besonders bevorzugt von 15 bis 24 nm und am meisten bevorzugt 18 bis 22 nm. Bei allen Partikelgrößen, die die Komponente (b) betreffen, handelt es sich um zahlengemittelte Werte (D50-Werte, d.h. 50 % der absoluten Anzahl an Partikeln haben einen Durchmesser kleiner als der angegebene D50-Wert).

Bei allen übrigen Partikelgrößen handelt es sich, wenn nicht anders angegeben, um volumengemittelten Partikelgrößen (D50-Werte, d.h. 50 % des Gesamtvolumens, das alle Partikel gemeinsam besitzen, ist in Partikeln enthalten, die einen Durchmesser kleiner als den angegebene Wert aufweisen). Die Partikelgrößenbestimmung im Bereich von 0,1 µm bis 1000 µm erfolgt vorzugsweise mittels statischer Lichtstreuung (SLS), beispielsweise mit einem statischen Laserstreuungs-Partikelgrößen-Analysator LA-960 (Horiba, Japan) oder mit einem Microtrac S100 Partikelgrößen-Analysator (Microtrac, USA). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße in einem Bereich von 1 nm bis 0,1 µm erfolgt vorzugsweise durch dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25°C, z.B. mit einem Malvern Zetasizer Nano ZS (Malvern Instruments, Malvern UK).

Im Fall aggregierter und agglomerierter Partikel kann die Primärteilchengröße anhand von TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf ein 50 Å dickes Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Es wurde gefunden, dass insbesondere Ytterbiumtrifluorid-Partikel mit einer Größe von kleiner 25 nm eine Erhöhung der Röntgenopazität der Werkstoffe ermöglichen, dabei aber nur einen geringen Effekt auf den Brechungsindex der Zusammensetzung haben. Anders als röntgenopake Gläser setzen sie somit nicht die Verwendung von Monomeren mit einem hohen Brechungsindex voraus, um eine gute Transluzenz zu gewährleisten. So ist es möglich Monomere mit niedrigem Brechungsindex und großem Brechungsindexwechsel zu verwenden.

Gemäß einer bevorzugten Ausführungsform sind die Ytterbiumtrifluorid-Partikel oberflächenmodifiziert. Hierzu werden sie vorzugsweise mit einer organischen Verbindung behandelt, die über funktionelle Gruppen verfügt, die an die Oberfläche der Ytterbiumtrifluorid-Partikel binden können. Bevorzugte funktionelle Gruppen sind Phosphat-, Phosphonat-, Carboxyl-, Dithiophosphat-, und Dithiophosphonatgruppen. Die Oberflächenmodifizierungsmittel weisen vorzugsweise außerdem radikalisch polymerisierbare Gruppen auf, die eine Vernetzung mit der organischen Komponente (a) ermöglichen.

Bevorzugte Oberflächenmodifizierungsmittel sind P-7,10,13,16-Tetraoxaheptadec-1-yl-phosphonsäure, P-[6-[2-[2-(2-Hydroxyethoxy)ethoxy]ethoxy]hexyl]phosphonsäure, 2,3-Di-(methacryloyloxy)-propyl-1-phosphonsäure, 2,3-Di-(methacryloyloxy)-propyl-1-bisphosphonsäure und 3-O-Benzyloxy-2-methacryloyloxy-propyl-1-bisphosphonsäure.

Die erfindungsgemäßen Dentalwerkstoffe enthalten vorzugsweise 1 bis 30 Gew.-%, besonders bevorzugt 3 bis 10 Gew.-% und ganz besonders bevorzugt 5 bis 10 Gew.-% nanoskalige röntgenopake Füllstoffe, insbesondere Ytterbiumtrifluorid-Partikel, bezogen auf die Masse des Dentalwerkstoffs.

### Anorganischer Füllstoff (c)

Bevorzugte **anorganische Füllstoffe (c)** sind Glaspulver, vorzugsweise Strontiumglaspulver und/oder zirkonhaltige Glaspulver. Ein ganz besonders bevorzugtes Glas ist das Glas mit der CAS-Nummer 65997-17-3. Die Glaspulver haben vorzugsweise eine mittlere Teilchengröße von 0,1 bis 1,5 µm, besonders bevorzugt 0,3 bis 1,2 µm und ganz besonders bevorzugt 0,4 bis 0,9 µm, am meisten bevorzugt 0.4 bis 0.8 µm, beispielweise 0,7 µm. Gröbere Teilchen mit einer Größe von 1,5 µm bis 10 µm, vorzugsweise 3 bis 9 µm, können gegebenenfalls in einer Gesamtmenge von 0 bis 15 Gew.-%, vorzugsweise 0 bis 10 Gew.-% bezogen auf die Gesamtmasse des Dentalwerkstoffes enthalten sein.

Die Verwendung von nanoskaligem Ytterbiumtrifluorid zur Erzielung von Röntgenopazität ermöglicht es, auf die Verwendung von Glaspulvern mit einem hohen Bariumgehalt zu verzichten. Diese haben einen relativ hohen Brechungsindex von 1,53. Glaspulver mit einem geringen Bariumgehalt, vorzugsweise von weniger als 20 Gew.-% (gemessen als BaO, bezogen auf die Masse des Glases), können jedoch eingesetzt werden. Diese weisen einen Brechungsindex von ca. 1,50 auf. Bariumfreie Glaspulver sind besonders bevorzugt.

Erfindungsgemäß bevorzugt sind weiterhin Gläser mit einem Brechungsindex unter 1,525, besonders bevorzugt unter 1,520 und ganz besonders bevorzugt unter 1,515. Vorzugsweise liegt der Brechungsindex der Gläser in einem Bereich von 1,490 bis 1,525, besonders bevorzugt 1,490 bis 1,520 und ganz besonders bevorzugt 1,49 bis 1,515. Wenn diese Gläser mit entsprechenden Monomermischungen kombiniert werden, ergeben sie überraschender Weise besonders gute Durchhärtungstiefen.

Anorganische Gläser werden vorzugsweise in einer Menge von 20 bis 80 Gew.-%, besonders bevorzugt 25 bis 70 Gew.-% und ganz besonders bevorzugt von 30 bis 60 Gew.-% eingesetzt, bezogen auf die Gesamtmasse des Dentalwerkstoffs.

Weitere bevorzugte anorganische Füllstoffe (c) sind SiO₂/ZrO₂-Mischoxide, beispielsweise mit einer volumengemittelten Primärteilchengröße von 2 bis 100 nm, bevorzugt 2 bis 60 nm, besonders bevorzugt 2 bis 40 nm und ganz besonders bevorzugt 3 bis 30 nm. Die Primärteilchen sind sphärisch und aggregiert zu Sekundärpartikeln mit einer Größe 0,5 bis 30 µm, bevorzugt 2 bis 25 µm, besonders bevorzugt von 3 bis 20 µm und ganz besonders bevorzugt 3 bis 15 µm. Sie lassen sich entsprechend US 8,617,306 B2 herstellen.

Durch die Zugabe des SiO₂/ZrO₂-Mischoxids kann die Polierbarkeit der erfindungsgemäßen Zusammensetzungen verbessert werden. Bevorzugt sind SiO₂/ZrO₂-Mischoxide mit einem Brechungsindex in einem Bereich von 1,490 bis 1,525, besonders bevorzugt 1,490 bis 1,520 und ganz besonders bevorzugt 1,49 bis 1,515. Das oder die SiO₂/ZrO₂-Mischoxide werden vorzugsweise in einer Menge von 1 bis 30 Gew.-%, besonders bevorzugt 2 bis 20 Gew.-% und ganz besonders bevorzugt 5 bis 15 Gew.-% eingesetzt, beispielsweise in einer Menge von 10 Gew.-%, bezogen auf die Gesamtmasse des Dentalwerkstoffs. Bevorzugt sind Mischoxide, die 23 Gew.-% oder weniger ZrO₂, besonders bevorzugt 1 bis 20 Gew.-% ZrO₂ und ganz besonders bevorzugt 5 bis 18 Gew.-% ZrO₂ enthalten.

Erfindungsgemäß sind solche Dentalwerkstoffe besonders bevorzugt, die als Füllstoff (c) eine Mischung mindestens eines Glaspulvers und mindestens eines SiO₂/ZrO₂-Mischoxids enthalten.

Zur Erzielung einer hohen Durchhärtungstiefe der erfindungsgemäßen Dentalwerkstoffe werden die Brechungsindices des Füllers (c) und der Monomerkomponente (a) wie oben beschrieben vorzugsweise aufeinander abgestimmt. Dabei wird die Monomerkomponente (a) bevorzugt auf einen Brechungsindex eingestellt, der identisch mit dem Brechungsindex des Füllstoffs (c) oder maximal 0,03 größer ist. Besonders bevorzugt ist der Brechungsindex der Monomerkomponente (a) 0,002 bis 0,02 und ganz besonders bevorzugt 0,005 bis 0,015 größer als der Brechungsindex des Füllstoffs (c).

Die erfindungsgemäßen Werkstoffe können als Füller (c) einen Füllstoff oder eine Füllstoffmischung enthalten. Bei der Verwendung von Füllstoffmischungen sind Werkstoffe bevorzugt, die als Komponente (c) überwiegend, d.h. zu mehr als 50 Gew.-%, bevorzugt zu mehr als 70 Gew.-% , besonders bevorzugt zu mehr als 80 Gew.-% bezogen auf die Gesamtmasse der Komponente (c), ganz besonders bevorzugt ausschließlich solche Füllstoffe enthalten, deren Brechungsindex in dem genannten Bereich liegt.

Der Brechungsindex ist ein stoffspezifischer Materialkennwert, der von der Wellenlänge des verwendeten Lichts, von der Temperatur, vom Druck und der Reinheit des Stoffes abhängt. Wenn nicht anders angegeben wird hier unter dem Begriff Brechungsindex in allen Fällen der bei Raumtemperatur mit Normlicht D65 gemessene Brechungsindex verstanden (n_{D}). Die Bestimmung des Brechungsindex flüssiger Monomere und Monomermischungen kann mit einem handelsüblichen Abbe-Refraktometer erfolgen.

Die Bestimmung des Brechungsindex (BI) von festen Stoffen, wie z.B. von anorganischen Füllstoffen oder Kompositfüllstoffen, erfolgt nach der Immersionsmethode. Die Stoffe werden bei Raumtemperatur (23°C) in Mischungen von Flüssigkeiten mit unterschiedlichen Brechungsindices dispergiert (sogenannte Immersionsflüssigkeiten). Dabei zeigen sich die Konturen der Feststoffpartikel umso deutlicher, je größer der Brechzahlunterschied zwischen Flüssigkeit und Feststoff ist. Ändert man nun die Brechzahl der Flüssigkeit so, dass sie der des Feststoffs näherkommt, werden die Partikelkonturen schwächer und verschwinden bei Angleichung der Brechindices völlig. Als Immersionsflüssigkeiten eignen sich Flüssigkeiten mit bekanntem Brechungsindex, z.B. Mischungen aus Benzylsalicylat (n_{D}²⁰ = 1,536) und Triacetin (n_{D}²⁰ = 1,431) oder Bromnaphthalin (n_{D}²⁰ = 1,657). Durch die Variation der Mengenverhältnisse dieser Stoffe kann der Brechungsindex der Mischung an den des zu messenden Feststoffs angepasst werden. Bei Übereinstimmung der Brechungsindices wird der Brechungsindex der Immersionsflüssigkeit mit einem Refraktometer bestimmt.

Zur Verbesserung des Verbundes zwischen den Füllstoffpartikeln und der Polymerisationsmatrix sind die Füllstoffe vorzugsweise oberflächenmodifiziert, besonders bevorzugt durch Silanisierung, ganz besonders bevorzugt mit radikalisch polymerisierbaren Silanen, insbesondere mit 3-Methacryloyloxypropyltrimethoxysilan. Zur Oberflächenmodifizierung von nicht-silikatischen Füllstoffen, z.B. von ZrO₂ oder TiO₂, können auch funktionalisierte saure Phosphate, wie z. B. 10-Methacryloyloxydecyldihydrogenphosphat eingesetzt werden.

### Kompositfüller (d)

Die erfindungsgemäßen Werkstoffe enthalten als **Komponente (d)** mindestens einen Kompositfüllstoff. Unter Kompositfüllstoffen werden Agglomerate bestehend aus anorganischen Füllstoffen verstanden, die durch eine Polymermatrix miteinander verbunden sind. Durch die Agglomeration der feinen anorganischen Füllstoffpartikel zu Kompositfüllstoffen mit einer durchschnittlichen Teilchengröße von 5 bis 100 µm, besonders bevorzugt 10 bis 60 µm und ganz besonders bevorzugt 15 bis 40 µm, sinkt die durch das radikalisch polymerisierbare Monomer (a) zu benetzende Oberfläche der Füllstoffpartikel, was den Monomergehalt senkt und sich somit beispielweise positiv auf den Volumenschrumpf und damit die Randqualität des Dentalwerkstoffs auswirkt.

Im Fall der Kompositfüllstoffe wird der Brechungsindex der gehärteten Polymermatrix vorzugsweise so gewählt, dass er mit dem Brechungsindex des darin enthaltenen anorganischen Füllstoffs übereinstimmt oder um maximal ± 0,2, vorzugsweise maximal ±0,1 und besonders bevorzugt maximal ± 0,01 davon abweicht, so dass die Partikel des Kompositfüllers eine hohe Transluzenz aufweisen. Wird mehr als ein anorganischer Füllstoff zur Herstellung des Kompositfüllers verwendet, weist vorzugsweise die überwiegende Menge der anorganischen Füllstoffe, d.h. mehr als 50 Gew.-%, besonders bevorzugt mehr als 80 Gew.-% bezogen auf die Masse der anorganischen Füllstoffe, einen Brechungsindex in dem genannten Bereich auf.

Die Kompositfüllstoffe werden vorzugsweise durch Härten von Kompositpasten hergestellt, die ein oder mehrere radikalisch polymerisierbare Monomere und einen oder mehrere anorganische Füllstoffe enthalten. Zur Herstellung der Kompositfüllstoffe sind die als Komponente (a) genannten Monomere, die als Komponenten (b) und (c) genannten Füllstoffe und die als Komponente (e) genannten Initiatoren bevorzugt. Solche Mischungen der Komponenten (a), (b), (c) und (e) zur Herstellung von Kompositfüllstoffen sind ebenfalls Gegenstand der Erfindung.

Besonders bevorzugte radikalisch polymerisierbare Monomere zur Herstellung der Kompositfüllstoffe sind Di(meth)acrylate, ganz besonders bevorzugt Glycerindimethayrylat (GDMA, BI = 1,477), Alkylendimethacrylate, besonders bevorzugt 1,10-Decanedioldimethacrylat (D₃MA, BI = 1,460) und Triethylenglykoldimethacrylat (TEGDMA, BI = 1,461), sowie Urethandimethacrylate, besonders bevorzugt RM3 und V837, und ganz besonders bevorzugt Urethandimethacrylate mit aromatischen Gruppen, insbesondere V380, und Mischungen davon. Am meisten bevorzugt sind D₃MA, V380, RM3 und Mischungen davon.

1,10-Decanedioldimethacrylat zeichnet sich durch einen besonders niedrigen Brechungsindex (BI) aus. Auch das Urethandimethacrylat RM3 zählt mit einem Brechungsindex von 1,485 zu den niedrig brechenden Monomeren. V380 weist mit 1,513 einen deutlich niedrigeren Brechungsindex auf als Bis-GMA mit 1,552, verfügt aber über dessen gute mechanische Wirkung auf das Komposit.

Bevorzugte Füllstoffe zur Herstellung des Kompositfüllers sind Gläser mit einem Brechungsindex unter 1,525, besonders bevorzugt unter 1,520 und ganz besonders bevorzugt unter 1,515. Vorzugsweise liegt der Brechungsindex der Gläser in einem Bereich von 1,490 bis 1,525, besonders bevorzugt 1,490 bis 1,520 und ganz besonders bevorzugt 1,49 bis 1,515. Besonders bevorzugt sind Strontiumgläser, bariumhaltige Gläser, vorzugsweise mit einem BaO-Gehalt von weniger als 20 Gew.-% (gemessen als BaO, bezogen auf die Masse des Glases), und/oder zirkonhaltige Glasfüllstoffe. Strontiumglasfüller sind besonders bevorzugt. Die Glasfüllstoffe haben vorzugsweise eine Partikelgröße von 0,4 bis 1 µm, wobei Strontiumglaspulver mit einer Partikelgröße von 0,4 bis 1 µm ganz besonders bevorzugt sind. Weitere besonders bevorzugte anorganische Füllstoffe sind die oben definierten SiO₂/ZrO₂-Mischoxide.

Zur Herstellung des Kompositfüllers (d) werden vorzugsweise anorganische Füllstoffe (c) verwendet, die einen Brechungsindex in einem Bereich von 1,490 bis 1,525, besonders bevorzugt 1,490 bis 1,520 und ganz besonders bevorzugt 1,49 bis 1,515 haben.

Erfindungsgemäß besonders bevorzugt sich Kompositfüller, die als anorganischen Füllstoff Ytterbiumtrifluorid enthalten, wobei das als Komponente (b) verwendete nanoskalige Ytterbiumtrifluorid bevorzugt ist. Ganz bevorzugt sind Ytterbiumtrifluoridpartikel mit einer zahlengemittelten Teilchengröße (D50-Wert) von < 25 nm. Der Kompositfüller enthält vorzugsweise 1 bis 20 Gew.-%, besonders bevorzugt 8 bis 13 Gew.-% Ytterbiumtrifluoridpartikel, bezogen auf die Gesamtmasse des Kompositfüllstoffs.

Der Kompositfüller kann vorteilhaft auch eine Mischung der genannten Gläser, Mischoxide und/oder Ytterbiumtrifluorid enthalten, besonders bevorzugt eine Mischung von Ytterbiumtrifluorid mit mindestens einem SiO₂/ZrO₂-Mischoxid.

Erfindungsgemäß bevorzugt sind Kompositfüller, die durch Polymerisation der folgenden Zusammensetzung erhältlich sind:
- 8 bis 50 Gew.-%, vorzugsweise 18 bis 50 Gew.-% radikalisch polymerisierbares Monomer,
- 1 bis 20 Gew.-%, vorzugsweise 8 bis 13 Gew.-% Ytterbiumtrifluoridpartikel, vorzugsweise mit einer mittleren Teilchengröße (zahlengemittelter D50-Wert) von < 25 nm,
- 40 bis 90 Gew.-%, vorzugsweise 50 bis 70 Gew.-% weitere anorganischer Füllstoffe, vorzugsweise SiO₂/ZrO₂-Mischoxid und
- 0,01 bis 2 Gew.-%, vorzugsweise 0,1 bis 1 Gew.-% Initiator für die radikalische Polymerisation.

Alle Prozentangaben beziehen sich, wenn nicht explizit anders angegeben auf die Gesamtmasse des Kompositfüllstoffs.

Die Zusammensetzungen können nach der Polymerisation gemahlen und als Pulver eingesetzt werden. Die Polymerisation erfolgt vorzugsweise thermisch oder photochemisch. Gemahlene Partikel haben in der Regel eine splitterförmige Form. Die gemahlenen Kompositfüller haben vorzugsweise eine mittlere Partikelgröße von 10 bis 50 µm, besonders bevorzugt von 10 bis 40 µm und ganz besonders bevorzugt von 30 bis 40 µm. Sie enthalten vorzugsweise maximal 10 Gew.-%, bezogen auf die Masse des gemahlenen Kompositfüllstoffs, an Partikeln mit einer mittleren Größe von < 10 µm. Bevorzugte Kompositfüller dieses Typs und Verfahren zu ihrer Herstellung werden in EP 1 234 567 A2 beschrieben.

Als Initiatoren für die thermische Polymerisation können die bekannten Peroxide wie Dibenzoylperoxid, Dilauroylperoxid, tert.-Butylperoctoat oder tert.-Butylperbenzoat eingesetzt werden, aber auch a,a'-Azo-bis(isobutyroethylester), Benzpinakol und 2,2'-Dimethylbenzpinakol sind geeignet. Bevorzugte thermische Initiatoren werden wie in der EP 1 234 567 A2 beschrieben.

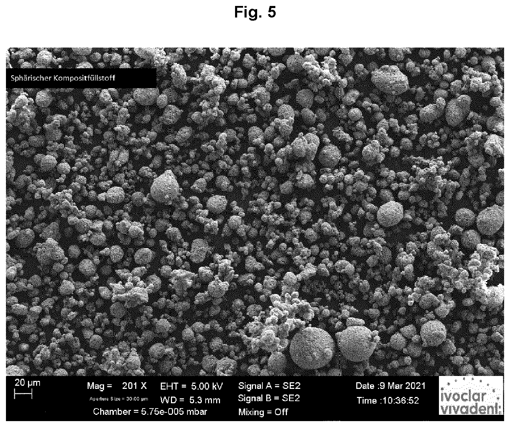

Gemäß einer besonders bevorzugten Ausführungsform haben die Partikel des Kompositfüllstoffs eine sphärische oder sphäroide Form (vergleiche Fig. 5). Diese Partikel können beispielsweise durch die sogenannte Inflightpolymerisation (Aerosol-Polymerisation) hergestellt werden. Hierzu wird das unpolymerisierte Ausgangsmaterial zur Herstellung des Kompositfüllers in Form kleiner Tröpfchen in eine Polymerisationskammer gesprüht und dann durch Bestrahlung mit Licht einer geeigneten Wellenlänge, bevorzugt im blauen Bereich, polymerisiert. Bei Bedarf kann die polymerisierbare Mischung vor dem Versprühen mit einem geeigneten Lösungsmittel verdünnt werden, um die Partikelgröße einzustellen. Das Lösungsmittel verdampft beim Versprühen und wirkt sich nicht auf die Zusammensetzung des Füllstoffs aus.

Als Initiatoren für die Lichthärtung eignen sich die als Komponente (e) genannten Photoinitiatoren, besonders 4,4'-Dichlorbenzil oder dessen Derivate sowie Campherchinon, vorzugsweise in Kombination mit einem Amin als Beschleuniger, wie zum Beispiel Ethyl-4-(dimethylamino)-benzoat, sowie Dibenzoylgermaniumderivate wie Bis-(4-methoxybenzoyl)-diethylgermanium.

Sphärische Kompositfüllstoffe können als anorganische Füllstoffe ebenfalls die oben genannten Stoffe enthalten, wobei hier Strontiumglasfüller, Ytterbiumtrifluorid, vorzugsweise nanoskaliges Ytterbiumtrifluorid, und/oder besonders die oben definierten SiO₂/ZrO₂-Mischoxide bevorzugt sind. Ganz besonders bevorzugt ist eine Mischung von nanoskaligem Ytterbiumtrifluorid und SiO₂/ZrO₂-Mischoxid. Das Strontiumglaspulver hat vorzugsweise eine Partikelgröße im Bereich von 0,4 bis 1 µm, besonders bevorzugt 0,5 bis 0,8 µm.

Der polymerisierte, sphärische bzw. sphäroide Kompositfüller hat vorzugsweise eine mittlere Partikelgröße von 5 bis 100 µm, besonders bevorzugt 10 bis 60 µm und ganz besonders bevorzugt 15 bis 40 µm.

Die Brechungsindices des Füllers (d) und der Monomerkomponente (a) werden vorzugsweise so aufeinander abgestimmt, dass der Brechungsindex der Komponente (a) dem Brechungsindex des Füllstoffs (d) entspricht oder maximal 0,025 größer ist. Vorzugsweise ist der Brechungsindex der Monomerkomponente (a) maximal bis 0,02, besonders bevorzugt maximal bis 0,01 größer als der Brechungsindex des Füllstoffs (d).

Die erfindungsgemäßen Werkstoffe können als Füller (d) einen Füllstoff oder eine Füllstoffmischung enthalten. Bei der Verwendung von Füllstoffmischungen sind Werkstoffe bevorzugt, die als Komponente (d) überwiegend, d.h. zu mehr als 50 Gew.-%, besonders bevorzugt zu mehr als 80 Gew.-%, jeweils bezogen auf die Gesamtmasse der Komponente (d), besonders bevorzugt ausschließlich solche Kompositfüllstoffe enthalten, deren Brechungsindices die genannte Bedingung erfüllen.

Kompositfüller (d) werden vorzugsweise in einer Menge von 5 bis 60 Gew.-%, besonders bevorzugt 15 bis 50 Gew.-% und ganz besonders bevorzugt von 15 bis 40 Gew.-% eingesetzt, bezogen auf die Gesamtmasse des Dentalwerkstoffs.

### Initiator für die radikalische Polymerisation (e)

Die erfindungsgemäßen Werkstoffe enthalten als **Komponente (e)** mindestens einen Initiator für die radikalische Polymerisation, vorzugsweise einen Photoinitiator.

Bevorzugte Photoinitiatoren sind Photosensibilisatoren, vor allem α-Diketone, wie 9,10-Phenanthrenchinon, 1-Phenyl-propan-1,2-dion, Diacetyl oder 4,4'-Dichlorbenzil oder deren Derivate, besonders bevorzugt Campherchinon (CC) und dessen Derivate, und Mischungen davon.

Die Photoinitiatoren werden vorzugsweise in Kombination mit Beschleunigern eingesetzt. Als Beschleuniger eignen sich besonders tertiäre Amine, wie z.B. tertiäre aromatische Amine, insbesondere N,N-Dialkyl-aniline, -p-toluidine oder -3,5-xylidine, p-(N,N-Dialkylaminophenylethanol, -benzoesäurederivate, -benzaldehyd, -phenylessigsäureester und -phenylpropionsäureester. Konkrete Beispiele hierfür sind N,N-Dimethylanilin, N,N-Dimethyl-p-toluidin, N,N,3,5-Tetramethylanilin, N,N-Dimethylamino-p-benzaldehyd, p-(Dimethylamino)-benzoesäureethylester oder p-(Dimethylamino)-benzonitril. Geeignet sind auch tertiäre aliphatische Amine, wie z.B. Tri-n-butylamin, Dimethylaminoethan-2-ol, Triethanolamin, Dimethylaminoethylmethacrylat, N,N-Dimethylbenzylamin, oder heterocyclische Amine, wie z.B. 1,2,2,6,6-Pentamethylpiperidin, und Aminosäure-Derivate, wie z.B. N-Phenylglycin. Alternativ können aminfreie Beschleuniger verwendet werden, wie z.B. Sulfinsäuren und Sulfinate, Borate, Enolate, Phosphine oder andere Verbindungen, die aktive Wasserstoffatome enthalten, z.B. heterocylische Verbindungen wie Morpholin-Derivate oder 1,3-Dioxolane.

Weitere erfindungsgemäß bevorzugte Photoinitiatoren sind Acyl- oder Bisacylphosphinoxide, insbesondere die in EP 0 007 505 A2, EP 0 073 413 A2, EP 0 184 095 A2 und EP 0 615 980 A2 beschriebenen Initiatoren. Bevorzugte Beispiele sind die kommerziell zugänglichen Verbindungen 2,4,6-Trimethylbenzoyldiphenylphosphinoxid (Lucirin^{™} TPO, BASF) und Bis(2,4,6-trimethylbenzoyl)phenylphosphinoxid (Irgacure^{™} 819, Ciba). Acyl- und Bisacylphosphinoxide gehören zur Gruppe der monomolekularen Photoinitiatoren und zeichnen sich durch eine geringe Eigenabsorption aus. Acyl- und Bisacylphosphinoxide können sich nachteilig auf die Durchhärtungstiefe und die Vickershärte in der Tiefe auswirken und werden daher vorzugsweise in einer Menge von weniger als 0,03 Gew.-% eingesetzt, ganz bevorzugt 0,01 Gew.-% oder weniger. In einer ganz bevorzugten Ausführungsform werden keine Acyl- oder Bisacylphosphinoxide verwendet.

Besonders bevorzugte Photoinitiatoren sind Acyl- oder Bisacylgermanium-Verbindungen, insbesondere die in der EP 1 905 413 A1 offenbarten Monoacyltrialkyl- und Bisacyldialkylgermanium-Verbindungen, wie z.B. Benzoyltrimethylgermanium, Bisbenzoyldiethylgermanium oder Bis(4-methoxybenzoyl)diethylgermanium. Acyl- und Bisacylgermanium-Verbindungen haben den Vorteil, dass sie sich nach der Bestrahlung entfärben (Bleaching Effekt) und so die Transmission der ausgehärteten Werkstoffe nicht beeinträchtigen. Außerdem handelt es sich um monomolekulare Photoinitiatoren, d.h. sie benötigen keinen Beschleuniger, um ihre volle Aktivität zu erreichen.

Photointiatoren können monomolekular (Norrish-Typ-I) oder bimolekular (Norrish-Typ-II) sein. Erfindungsgemäß wird vorzugsweise eine Kombination mindestens eines monomolekularen und mindestens eines bimolekularen Photoinitiators verwendet. Solche Kombinationen werden z.B. in der EP 3 659 575 A1 beschrieben. Besonders bevorzugt werden der mindestens eine monomolekulare Photoinitiator und der mindestens eine bimolekulare Photoinitiator in einem Verhältnis von 1,4:1 bis 1:2, bezogen auf ihre Gewichtsanteile, eingesetzt.

Bevorzugte monomolekulare Photoinitiatoren sind Monoacyl- oder Bisacylphosphinoxide, Diacyldialkylgermanium- und Tetraacylgermanium-Verbindungen sowie Tetraacylstannane. Besonders bevorzugte monomolekulare Photoinitiatoren sind 2,4,6-Trimethylbenzoyldiphenylphosphinoxid, Bis(2,4,6-trimethylbenzoyl)-phenylphosphinoxid, Dibenzoyldiethylgerman, Bis(4-methoxybenzoyl)diethylgerman (MBDEGe, Ivocerin^{®}), Tetrabenzoylgerman, Tetrakis(o-methylbenzoyl)german, Tetrakis(mesitoyl)stannan und Mischungen davon.

Bevorzugte bimolekulare Photoinitiatoren sind α-Diketone oder deren Derivate. Besonders geeignete α-Diketone oder Derivate davon sind Campherchinon (CQ), 9,10-Phenanthrenchinon, 1-Phenyl-propan-1,2-dion, 2,2-Dimethoxy-2-phenyl-acetophenon, Diacetyl oder 4,4'-Dichlorbenzil oder deren Derivate. Ganz besonders bevorzugt sind Campherchinon (CQ), 2,2-Dimethoxy-2-phenyl-acetophenon und Mischungen davon. Am meisten bevorzugt sind α-Diketone in Kombination mit Aminen als Reduktionsmittel, wie z.B. 4-(Dimethylamino)-benzoesäureester (EDMAB), N,N-Dimethylaminoethylmethacrylat, N,N-Dimethyl-sym.-xylidin, Triethanolamin und Mischungen davon. Vorzugsweise wird ein Verhältnis von bimolekularem Photoinitiator zu Amin von 1: 1 bis 1:6, bezogen auf ihre Gewichtsanteile, eingesetzt.

Besonders bevorzugte Kombinationen mindestens eines monomolekularen Photoinitiators, mindestens eines bimolekularen Photoinitiators und mindestens eines Amins als Reduktionsmittel sind Mischungen, die als monomolekularen Photoinitiator Bis(4-methoxybenzoyl)diethylgerman (MBDEGe, Ivocerin^{®}), Bis(2,4,6-trimethylbenzoyl)phenylphosphinoxid, Tetrakis-(o-methylbenzoyl)german, Tetrakis(mesitoyl)stannan oder eine beliebige Mischung davon, als bimolekularen Photoinitiator Campherchinon (CQ) und/oder 2,2-Dimethoxy-2-phenyl-acetophenon und als Reduktionsmittel 4-(Dimethyl-amino)benzoesäureester (EDMAB) enthalten. Ganz besonders bevorzugt wird eine Mischung aus Bis(4-methoxybenzoyl)diethylgerman (MBDEGe, Ivocerin^{®}) und Campherchinon und 4-(Dimethyl-amino)benzoesäureester (EDMAB) eingesetzt.

Initiatoren werden vorzugsweise in einer Menge von 0,005 bis 3,0 Gew.-%, besonders bevorzugt 0,01 bis 2,0 Gew.-%, besonders bevorzugt 0,1 bis 1 Gew.-% eingesetzt, bezogen auf die Gesamtmasse des Dentalwerkstoffs. Diesen Mengenangaben schließen alle Initiatorbestandteile, wie z.B. auch Beschleuniger, ein.

Erfindungsgemäße Zusammensetzungen, die einen der genannten Initiatoren enthalten, können beispielsweise durch Bestrahlen mit Blaulicht (Wellenlängenbereich von 400 bis 500 nm) gehärtet werden, vorzugweise durch Bestrahlen mit einer LED-Lampe mit einer Leistung zwischen 1200±120 mW/cm² und 3050±305 mW/cm².

### Farbmittel

Die erfindungsgemäßen Werkstoffe werden vorzugsweise mit Farbstoffen und/oder Farbpigmenten eingefärbt. Gemäß der 16 Farben der VITA classical A1-D4^{®} Farbskala können die natürlichen Zähne in vier Gruppen eingeteilt werden (rötlich-bräunlich, rötlich-gelblich, Grautöne, rötlich-grau). Zur Erzielung einer natürlichen Färbung werden vorzugsweise rote, gelbe, schwarze und/oder weiße Farbstoffe und/oder Farbpigmente miteinander kombiniert, wobei pro Farbton ein oder mehrere Farbstoffe und/oder Pigmente verwendet werden können.

Erfindungsgemäß bevorzugte Pigmente sind anorganische Pigmente, wie Oxide, Oxid-Hydrate, besonders bevorzugt sind Metalloxid-, Halbmetalloxid- und Metalloxid-Hydrat-Pigmente. Ganz besonders bevorzugte Pigmente sind die Oxide der Elemente Eisen, Titan und Silizium, sowie die Oxid-Hydrate des Elements Eisen.

Bevorzugte organische Farbstoffe sind Farbstoffe, die mindestens eine cyklische aromatische C₆-Einheit enthalten, und Azofarbstoffe. Besonders bevorzugt sind Azofarbstoffe, die mindestens eine cyklische aromatische C₆-Einheit enthalten.

Erfindungsgemäß werden vorzugsweise mindestens 3, vorzugsweise 3 bis 10, besonders bevorzugt, 3 bis 9 und ganz besonders bevorzugt 3 bis 7 unterschiedliche Farbpigmente und/oder Farbstoffe verwendet, die gemäß einer besonders bevorzugten Ausführungsform aus den oben genannten Farbstoffen und Pigmenten, vorzugsweise Pigmenten, ausgewählt sind.

### Weitere Bestandteile

Die erfindungsgemäßen Zusammensetzungen können außerdem weitere **Additive** enthalten, vor allem Rheologiemodifizierungsmittel, Stabilisatoren, wie z.B. Polymerisationsstabilisatoren, antibakterielle Wirkstoffe, fluoridionenabgebende Additive, optische Aufheller, Fluoreszenzmittel, UV-Absorber, Stoffe zur Verbesserung der Bruchzähigkeit und/oder Effektmittel. Die Gesamtmenge an Additiven beträgt vorzugsweise maximal 4 Gew.-%, besonders bevorzugt maximal 3 Gew.-%, bezogen auf die Gesamtmasse des Werkstoffs.

Die erfindungsgemäßen Dentalwerkstoffe enthalten vorzugsweise:
- 5 bis 40 Gew.-%, vorzugsweise 10 bis 35 Gew.-%, besonders bevorzugt 12 bis 30 Gew.-% mindestens eines radikalisch polymerisierbaren Monomeren (a),
- 1 bis 30 Gew.-%, vorzugsweise 3 bis 10 Gew.-%, besonders bevorzugt 5 bis 10 Gew.-% Ytterbiumtrifluoridpartikel (b) mit einer mittleren Teilchengröße (zahlengemittelter D50-Wert) von 25 nm oder weniger,
- 20 bis 90 Gew.-%, vorzugsweise 30 bis 70 Gew.-%, besonders bevorzugt 40 bis 65 Gew.-% anorganischen Füllstoff (c),
- 5 bis 60 Gew.-%, vorzugsweise 15 bis 50 Gew.-%, besonders bevorzugt 15 bis 40 Gew.-% Kompositfüllstoff (d) und
- 0,005 bis 3,0 Gew.-%, vorzugsweise 0,01 bis 2,0 Gew.-%, besonders bevorzugt 0,1 bis 1 Gew.-% Initiator für die radikalische Polymerisation (e).

Besonders bevorzugt sind Dentalwerkstoffe, die folgende Zusammensetzung aufweisen:
- 15 bis 25 Gew.-% radikalisch polymerisierbare Monomeren (a),
- 5 bis 10 Gew.-% Ytterbiumtrifluoridpartikel (b),
- 45 bis 55 Gew.-% anorganischen Füllstoff (c),
- 15 bis 40 Gew.-% Kompositfüllstoff (d) und
- 0,1 bis 0,5 Gew.-% Initiator für die radikalische Polymerisation (e).

Wenn nicht explizit anders angegeben, beziehen sich die Prozentangaben jeweils auf die Gesamtmasse des Dentalwerkstoffs.

Die für Komponente (b) gemachten Mengenangaben schließen nicht das in Komponente (d) enthaltene Ytterbiumtrifluorid ein. Die erfindungsgemäßen Dentalwerkstoffe enthalten vorzugsweise insgesamt, d.h. in den Komponenten (b) und (d), 2 bis 30 Gew.-%, besonders bevorzugt 3 bis 20 Gew.-% und ganz besonders bevorzugt 4 bis 12 Gew.-% nanoskaliges Ytterbiumtrifluorid, bezogen auf die Gesamtmasse des Werkstoffs.

Nanoskalige Füllstoffen haben häufig eine hohe Verdickungswirkung, die sich nachteilig auf die Herstellbarkeit und die Eigenschaften von Dentalwerkstoffen auswirken kann und die die Füllstoffmenge und im Fall von röntgenopaken Füllstoffen damit auch die maximal erzielbare Röntgenopazität begrenzt. Größere Partikel haben zwar eine geringere Verdickungswirkung, wirken sich aber besonders bei Ytterbiumtrifluorid nachteilig auf die optischen Eigenschaften und insbesondere auf die Durchhärtungstiefe der Materialien aus. Erfindungsgemäß wurde gefunden, dass eine hohe Durchhärtungstiefe in Kombination mit einer guten Röntgenopazität insbesondere dann erzielt werden kann, wenn ein Teil des eingesetzten nanoskaligen Ytterbiumtrifluorids im Kompositfüller (d) enthalten ist, d.h. in agglomerierter Form vorliegt. Durch diese Aufteilung und die Verwendung von Ytterbiumtrifluorid mit der erfindungsgemäßen Partikelgröße können eine hohe Durchhärtungstiefe und Röntgenopazität erzielt und gleichzeitig die Verdickungswirkung des Füllstoffs minimiert werden.

Naturgemäß sind solche Werkstoffe bevorzugt, bei denen die Komponenten (a) bis (e) aus den oben definierten bevorzugten und besonders bevorzugten Materialen ausgewählt sind.

Die erfindungsgemäßen Dentalwerkstoffe enthalten vorzugsweise insgesamt 30 bis 95 Gew.-%, besonders bevorzugt 50 bis 90 Gew.-% und ganz besonders bevorzugt 65 bis 85 Gew.-% an Füllstoffen (Komponenten (b), (c) und (d)), bezogen auf die Gesamtmasse des Dentalwerkstoffs.

Erfindungsgemäß sind weiterhin solche Dentalwerkstoffe bevorzugt, in denen der Füllstoff (c) bariumfrei ist, die als Initiator (e) Acyl- und Bisacylphosphinoxide in einer Menge von weniger als 0,03 Gew.-% enthalten, bevorzugt 0,01 Gew.-% oder weniger, und/oder die als Monomer (a-4) mindestens ein monofunktionelles radikalisch polymerisierbares Monomer enthalten, vorzugsweise in einer Menge von weniger als 2 Gew.-%, besonders bevorzugt in einer Menge von 0,1 bis 1,9 Gew.-%, ganz besonders bevorzugt 0,2 bis 1,8 Gew.-% und am meisten bevorzugt 1,3 bis 1,7 Gew.-%, bezogen auf die Gesamtmasse des Dentalwerkstoffs, wobei Dentalwerkstoffe, die diese drei Merkmale gleichzeitig aufweisen, besonders bevorzugt sind.

Die erfindungsgemäßen Dentalwerkstoffe zeichnen sich durch ein zur Restauration geschädigter Zähne sehr vorteilhaftes Eigenschaftsprofil aus. Sie haben einerseits eine hohe Röntgenopazität und gute mechanische Eigenschaften und andererseits hervorragende ästhetische Eigenschaften sowie eine hohe Durchhärtungstiefe.

Eine hohe Durchhärtungstiefe ermöglicht das Härten von großen Schichtdicken mit Licht und vereinfacht so die Handhabung der Werkstoffe erheblich. Die Werkstoffe eignen sich besonders als Bulk-Fill-Komposite.

Die Durchhärtungstiefe wird gemäß der DIN EN ISO 4049:2018-04 bestimmt und beträgt vorzugsweise 3 mm oder mehr, besonders bevorzugt 3,5 bis 5 mm. Besonders vorteilhaft ist, dass sich diese Durchhärtungstiefen bei den erfindungsgemäßen Werkstoffen mit einer kurzen Belichtungszeit von nur 3 Sekunden (bei 3050±305 mW/cm²) realisieren lassen. Die erfindungsgemäßen Werkstoffe erlauben die Herstellung auch großer Zahnfüllungen mit nur 1 bis 2 Schichten. Dies ermöglicht ein effizientes Arbeiten im Seitenzahnbereich.

Darüber hinaus eignen sich die erfindungsgemäßen Dentalwerkstoffe aufgrund ihrer ästhetischen Eigenschaften auch hervorragend für Frontzahnrestaurationen. Die Herstellung ästhetisch überzeugender Dentalrestaurationen ist mit nur einem Material möglich, und es ist nicht erforderlich, mehrere Materialien zu verwenden und aufeinander abzustimmen. Die vorteilhaften ästhetischen Eigenschaften werden maßgeblich durch das Einstellen eines bestimmten Verhältnisses von Kontrastwert (CR-Wert) zu Transmission erreicht. Die erfindungsgemäßen Dentalwerkstoffe weisen vorzugsweise einen CR-Wert von 55 bis 75, besonders bevorzugt von 60 bis 70 und ganz besonders bevorzugt von 62 bis 68 auf. Die Transmission der eingefärbten Werkstoffe liegt vorzugsweise zwischen 8 und 25 %, besonders bevorzugt zwischen 9 und 22 % und ganz besonders bevorzugt zwischen 10 und 18 %. Alle Angaben beziehen sich auf die gehärteten Materialien.

Unter dem CR-Wert versteht man das Verhältnis der Farbmessung vor einem weißen und einem schwarzen Hintergrund. Der Kontrastwert CR (inklusive der L, a und b-Werte) wird gemäß BS 5612 (Britischer Standard) unter Verwendung eines Spektralkolorimeters (z.B. Minolta CM-3700d) bestimmt. Die Bestimmung des Kontrastwerts besteht aus zwei Einzelmessungen. Das zu analysierende Teststück wird hierfür vor einem schwarzen Keramikkörper mit einer Reflektion von maximal 4 % und entsprechend vor einem weißen Keramikkörper mit einer Reflektion von minimal 86 % angeordnet und diese dann kolorimetrisch analysiert. Bei Verwendung von hochtransparenten Teststücken wird Reflektion/Absorption hauptsächlich durch den keramischen Hintergrund hervorgerufen, wohingegen die Reflektion durch das Teststück hervorgerufen wird, wenn ein opakes Material verwendet wird. Das Verhältnis von reflektiertem Licht vor schwarzem Hintergrund zu reflektiertem Licht vor weißem Hintergrund ist das Maß für den Kontrastwert, wobei vollständige Transmission zu einem Kontrastwert von 0 und vollständige Opazität zu einem Kontrastwert von 100 führt.

Das Zusammenspiel von CR-Wert und Transmission ergibt Werkstoffe mit herausragen optischen Eigenschaften. Eine Transmission im erfindungsgemäßen Bereich erlaubt es dem Umgebungslicht in Werkstoff einzudringen und lässt ihn lebendig erscheinen. Gleichzeitig wird in Werkstoffen mit einem erfindungsgemäßen CR-Wert die in den Werkstoff einstrahlende Farbe der umgebenden Zahnhartsubstanz so gebrochen, dass der Werkstoff eine ähnliche Farbe wie die Zahnhartsubstanz zu haben scheint.

Die Messung der Transmission erfolgt auf die in den Ausführungsbeispielen beschriebene Weise an runden Prüfkörpern mit einem Durchmesser von 20 mm und einer Höhe von 1 mm.

Aufgrund dieser Eigenschaften können die erfindungsgemäßen Werkstoffe mit wenigen Farben den Farbraum der natürlichen Zahnfarben vollständig abdecken, der üblicherweise die 16 Farben der VITA classical A1-D4^{®} Farbskala umfasst. Bei den erfindungsgemäßen Werkstoffen deckt jede Farbe aufgrund des definierten CR-Werts und der definierten Transmission in Kombination ihrer spezifischen Farb- und Helligkeitseinstellung mehrere Farben der üblichen 16 Farben ab. Die Werkstoffe integrieren sich ideal in den natürlichen Zahn, da sie einerseits die Farbe der umgebenden Zahnhartsubstanz aufnehmen und gleichzeitig farbig und opak genug sind, um einen gräulichen Eindruck zu vermeiden.

Ein besonderer Vorteil der erfindungsgemäßen Dentalwerkstoffe ist außerdem, dass sie aufgrund der optimal aufeinander abgestimmten Bestandteile des Dentalwerkstoffs eine hohe Glanzstabilität in Kombination mit einer guten Polierbarkeit und einer geringen Oberflächenrauheit haben, was sich nicht nur positiv auf die ästhetischen Eigenschaften sondern auch auf die Gebrauchseigenschaften auswirkt.

Die Oberflächentextur eines Restaurationsmaterials ist nicht nur aus ästhetischen Gründen wichtig, sondern sie hat darüber hinaus auch ein wesentlicher Einfluss auf die Plaqueakkumulation, Verfärbung und Verschleiß der Restauration. Eine zunehmende Rauheit ist korreliert mit erhöhter Plaqueakkumulation und Verfärbung. Daher ist das Finieren und Polieren ein wichtiger Schritt bei der Herstellung sowohl von direkten als auch von indirekten Restaurationen. Die erfindungsgemäßen Dentalwerkstoffe ergeben eine besonders vorteilhafte Oberflächenrauheit nach dem Polieren. Die Oberflächenrauheit von Restaurationsmaterialien ändert sich im Verlauf der Gebrauchszeit. Beispielsweise bewirkt bei Kompositwerkstoffen die Zahnreinigung mit Zahnbürste und Zahnpaste einen Materialabtrag und eine Veränderung der Oberflächenrauheit. Die erfindungsgemäßen Werkstoffe zeichnen sich hier durch eine besondere Widerstandsfähigkeit aus.

Die erfindungsgemäße Werkstoffe haben eine Glanzstabilität (nach 1 Stunde putzen) von 30 GE oder mehr, vorzugsweise von 35 bis 95 GE, ganz bevorzugt von 40 bis 90 GE. Sie weisen eine Polierbarkeit von 40 GE oder mehr auf, vorzugsweise von 60 bis 90 GE, ganz bevorzugt von 70 bis 80 GE. Sie haben zudem eine Oberflächenrauheit von 0,40 µm oder weniger, vorzugsweise von 0,01 bis 0,30 µm, ganz bevorzugt von 0,02 bis 0,20 µm, wobei Werkstoffe mit einer Oberflächenrauheit von 0,03 bis 0,175 µm und insbesondere 0,04 bis 0,15 µm besonders bevorzugt sind.

Zur Bestimmung der Glanzstabilität der Dentalwerkstoffe wird das zu testende Material in eine zylindrische Metallform (d = 10 mm, h = 5-10 mm) gefüllt (n=8 pro Serie). Das Material wird anschließend 3x für 30s mit Licht im Wellenlängenbereich von 380 bis 520 nm (Lichtleistung 1100 mW/cm²) belichtet. Die gehärteten Prüfkörper werden dann auf einen REM-Träger geklebt und für 350s mit Licht im Wellenlängenbereich von 380 bis 520 nm (Lichtleistung 1200 mW/cm²) endpolymerisiert. Nachdem die Prüfkörper trocken für 24 h bei 37°C gelagert wurden, werden sie mit einer Poliermaschine (Phoenix 4000, Buehler) unter Verwendung von Schleifpapier (4000 grit-Schleifpapier, Buehler) und Polierflüssigkeit (Aluminiumoxidpartikel, Partikelgröße 0,05 Mikrometer, Buehler) auf Hochglanz poliert. Die so präparierten Prüfkörper werden mittels eines Zahnbürstensimulationsgerätes (Willytec, SD-Mechatronik, Feldkirchen-Westerham, Deutschland; Zahnbürstenköpfe von Eco-logic, Trisa, Schweiz) einer einstündigen kreisförmigen Zahnbürstenbewegung (Auflagekraft 50 g) in einem Zahnpastenslurry (Zahnpasta Signal Anti-Karies, 250 mL Zahnpaste pro 1L Wasser) ausgesetzt. Nach jeweils 15 min, 30 min, 45 min und 60 min Zahnbürstensimulation wird der Oberflächenglanz mit einem Glanzmessgerät (Novo-curve, Rhopoint, Bexhill-on-Sea, GB) bestimmt. Das Messprinzip des Glanzmessgeräts besteht darin, dass im Winkel von 60° ein Lichtstrahl auf die zu messende Oberfläche auftrifft und die Intensität des reflektierten Lichtes im Gerät gemessen und mit einem Referenzwert verglichen wird. Je höher der Wert ist, desto höher ist der Glanz. Der Glanz wird in Glanzeinheiten (GE) in Relation zu einem Kalibrierglas (schwarze Glasplatte mit einem Wert von 93,7 GE) gemessen. Schwarzer Schaumstoff hat einen Wert 0 GE. Das handelsübliche Material Tetric EvoCeram (2018) erreicht beispielsweise einen Wert von 45 GE.

Die Polierbarkeit der Dentalwerkstoffe wird folgendermaßen geprüft: Jeder Prüfkörper (n=8 pro Serie) wird mit einem Polierer (Einschritt-Polierer OptraGloss HP) nach standardisierter Aufrauhung (320 grit Schleifpapier, Buehler) mit einem definierten Anpressdruck von 2 N poliert. Die Politur wird unter Wasserzufuhr mit einem zahnärztlichen Handstück (KaVo) und einer Umdrehungszahl von 10 000 U/min durchgeführt. Jeder Prüfkörper wird für 10 s/20 s/30 s poliert. Nach jedem Polierintervall wird der Prüfkörper mit Wasserspray abgespült, mit dem Luftbläser getrocknet und sowohl der Oberflächenglanz als auch die Oberflächenrauheit gemessen. Der durch den Polierer erzielte Glanz wird in Glanzeinheiten (GE, analog zur Beschreibung der Glanzstabilität) mit dem oben beschriebenen Glanzmessgerät gemessen.

Zur Messung der Oberflächenrauheit Ra (mittlere Oberflächenrauheit Ra in µm) werden die Proben mit einem speziellen optischen Sensor (CWL) mit fokussiertem Weißlicht beleuchtet. Eine interne optische Vorrichtung, deren Brennweite eine starke Wellenlängenabhängigkeit aufweist, spaltet das weiße Licht in verschiedene Farben (entsprechend den unterschiedlichen Wellenlängen) auf. Ein miniaturisiertes Spektrometer detektiert die Farbe des von der Probe reflektierten Lichts und bestimmt mit Hilfe einer internen Kalibriertabelle die vertikale Position auf der Probenoberfläche. Die xy-Position wird mit Drehgebern gemessen. Die Messung erfolgt mit einer Auflösung von 10 nm in der Höhe und 1 bis 2 µm lateraler Auflösung bei einer maximalen Messfrequenz von 1000 Hz. Die mittlere Rauheit Ra (in µm) ist das arithmetische Mittel der Profil-Ordinaten. Die Messung erfolgt vorzugsweise mit dem Messgerät FRT MicroProf (FRT MicroProf, Fries Research & Technology GmbH, Bergisch Gladbach, DE).

Die erfindungsgemäßen Dentalwerkstoffe weisen nach der Aushärtung eine Biegefestigkeit von mindestens 100 MPa, vorzugsweise von 100 bis 200 MPa, besonders bevorzugt 100 bis 180 MPa und ganz besonders bevorzugt von 100 bis 160 MPa auf, haben in 4,0 mm Tiefe eine Vickershärte von 80% oder mehr im Vergleich zur Oberflächenhärte (bei 0,5 mm), eine Transmission von 10-18%, eine Glanzstabilität von vorzugsweise 30 bis 95 GE, besonders bevorzugt von 35 bis 90 GE und ganz besonders bevorzugt von 40 GE bis 90 GE, eine Polierbarkeit von vorzugsweise 50 bis 95 GE, besonders bevorzugt von 60 bis 90 GE und ganz besonders bevorzugt von 70 GE bis 90 GE, und eine Oberflächenrauheit von vorzugsweise 0,20 µm oder weniger, besonders bevorzugt 0.20 bis 0.01 µm und ganz besonders bevorzugt von 0.15 bis 0.01 µm. Sie zeichnen sich weiterhin dadurch aus, dass sie sich in kurzer Zeit, nämlich vorzugsweise in 3 bis 10 s mit Licht im Wellenlängenbereich von 380 bis 520 nm (Intensitätsspitzen bei 410 und 460 nm) und einer Intensität von 1890 bis 3355 mW/cm² ausgehärtet werden können. Aufgrund dieser Kombination von Eigenschaften eignen sie sich sowohl zur Restauration von Frontzähnen als auch von Seitenzähnen.

Die erfindungsgemäßen Dentalwerkstoffe haben zudem eine hohe Röntgenopazität. Eine hohe Röntgenopazität ermöglicht eine eindeutige Unterscheidung von der natürlichen Zahnsubstanz. Die Röntgenopazität wird nach der ISO-Norm 4049 bestimmt. Dazu wird ein Prüfkörper aus dem polymerisierten Dentalwerkstoff zusammen mit einer Aluminiumtreppe mit einer Stufenhöhe von 1 mm mit einer Röntgenkamera aufgenommen. Man vergleicht den Schwärzungsgrad der Bilder und gibt die Röntgenopazität in % Al an, 100% Röntgenopazität entsprechen dem Schwärzungsgrad von 1 mm Aluminium. Die erfindungsgemäßen Werkstoffe haben vorzugsweise eine Röntgenopazität von 140 % bis 350 % Al, besonders bevorzugt von 160 % bis 250 % Al.

Die erfindungsgemäßen Dentalwerkstoffe zeichnen sich durch ein vorteilhaftes Eigenschaftsprofil aus, d.h., sie erzielen in allen der genannten Eigenschaften Werte, die nach dem derzeitigen Stand der Technik von Dentalwerkstoffen erwartet werden. Dies ermöglicht es, mit nur einem Werkstoff Dentalrestaurationen und insbesondere Zahnfüllungen herzustellen, die dem derzeitigen medizinischen Kenntnisstand entsprechen.

Die erfindungsgemäßen Werkstoffe weisen eine hohe Standfestigkeit und eine geringe Klebrigkeit auf und sind stopfbar und modellierbar. D.h., sie lassen sich ähnlich wie Amalgam verarbeiten und in Zahnkavitäten einbringen, verdichten und in eine zur restlichen Zahnhartsubstanz passende Form modellieren. Sie eignen sich daher hervorragend als Zahnfüllungsmaterialien, insbesondere für direkte und indirekte Front- und Seitenzahnfüllungen aller Klassen. Diese Eigenschaften werden durch die erfindungsgemäße Wahl von Monomeren, Füllstoffart und Füllstoffmenge erzielt.

Unter dem Begriff "modellierbar" werden hier Werkstoffe verstanden, die eine Viskosität von vorzugsweise 10 bis 500 kPa·s, besonders bevorzugt 20 bis 450 kPa·s und ganz besonders bevorzugt 50 bis 400 kPa·s aufweisen. Die hier beschriebenen Viskositäten wurden wie folgt gemessen: 110 mg des zu bestimmenden Materials werden auf ein 12 mm-Platte/Platte Messystem eines Rotationsviskosimeters (z.B. "MCR 302" der Firma Anton Paar) gegeben. Die Temperatur wird auf 23°C und der Spaltabstand zwischen den Platten auf 0,5 mm eingestellt. Die Proben werden nach dem Auftragen nicht getrimmt. Die Probe wird für 2 Minuten (Ruhephase) temperiert, anschließend mit einer Scherrate von 0,01 s⁻¹ für eine Minute geschert. Schließlich erfolgt die Messung von 3 Datenpunkten mit je einer Minute Scherung bei der Scherrate von 0,01 s⁻¹. Als Ergebnis wird der Mittelwert dieser Datenpunkte ausgegeben.

Die erfindungsgemäßen Dentalwerkstoffe eignen sich primär zur intraoralen Anwendung durch den Zahnarzt zur Restauration geschädigter Zähne (therapeutische Anwendung), insbesondere als dentale Zemente, Beschichtungs- oder Verblendmaterialien und ganz besonders als Füllungskomposite und als sogenannte Bulk-Fill-Komposite.

Die erfindungsgemäßen Werkstoffe können auch extraoral (nicht therapeutisch) eingesetzt werden, beispielsweise bei der Herstellung oder Reparatur von Dentalrestaurationen (nicht therapeutische Anwendung). Sie eignen sich insbesondere als Materialien zur Herstellung von Inlays, Onlays, Kronen oder Brücken.

Die Erfindung wird im Folgenden anhand von Figuren und Ausführungsbeispielen näher erläutert:
Fig. 1 zeigt einen humanen Molaren mit linkseitiger erfindungsgemäßer Restauration und rechtsseitiger Restauration mit einem handelsüblichen Kompositmaterial (Omnichroma, Fa. Tokuyama Dental).
Fig. 2 zeigt einen humanen Frontzahn mit einer linksseitigen Restauration mit einem handelsüblichen ästhetischen Kompositmaterial (Tetric EvoCeram, Fa. Ivoclar Vivadent AG) und einer rechtsseitigen großen Klasse 3 Kavität.
Fig. 3 zeigt den humanen Frontzahn aus Fig. 2 mit einer linksseitigen Restauration mit einem handelsüblichen ästhetischen Kompositmaterial (Tetric EvoCeram, Fa. Ivoclar Vivadent AG) und einer rechtsseitigen Restauration mit dem erfindungsgemäßen Werkstoff aus Beispiel 4 eingefärbt für dunkle Zahnfarben. Beide Restaurationen fügen sich gut in den Zahn ein und sind praktisch nicht zu erkennen.
Fig. 4 zeigt den humanen Frontzahn aus Fig. 2 mit einer linksseitigen Restauration mit einem handelsüblichen ästhetischen Kompositmaterial (Tetric EvoCeram, Fa. Ivoclar Vivadent AG) und einer rechtsseitigen Restauration mit dem erfindungsgemäßen Werkstoff aus Beispiel 6 eingefärbt für dunkle Zahnfarben. Beide Restaurationen fügen sich gut in den Zahn ein und sind praktisch nicht zu erkennen.
Fig. 5 zeigt eine rasterelektronenmikroskopische Aufnahme der sphärischen Partikel aus Beispiel 1 in 200-facher Vergrößerung.

### Ausführungsbeispiele

Mit den in den folgenden Ausführungsbeispielen angegebenen Rezepturen wurden dentale Dentalwerkstoffe hergestellt und wie beschrieben untersucht. Die Komponenten wurden mit einem Magnetrührer, einem Kneter (Firma Linden, Maschinentyp LPM 0.5 SP) bzw. mit einem Zentrifugalmischer (Speedmixer DAC 600.2 der Firma Hausschild) miteinander gemischt.

Zur Bestimmung der Transmission der Werkstoffe wurden gehärtete, runde Prüfkörper (Durchmesser: 20 mm, h = 1 mm) hergestellt und farbmetrisch mit Hilfe eines Spektralphotometers mit Normlicht D65 (z.B. Spectrophotometer CM-5, Minolta) vermessen. Als Referenzmessung für 0% Transmission wird der Strahlengang mit der zum Gerät gehörenden, "Nullkalibrierplatte" vollständig versperrt. Die Referenzmessung für 100% Transmission wird durchgeführt, indem keine Probe eingesetzt wird, der Strahlengang somit leer ist. Die Polymerisation des Prüfkörpers erfolgt mit einer LED- Lampe (3 s bei 3050±305 mW/cm²).

Die Messung von Biegefestigkeit und Durchhärtungstiefe erfolgte nach ISO 4049:2009: Zahnheilkunde - Polymerbasierende Restaurationsmaterialien. Dabei entspricht der angegebene Wert der Durchhärtungstiefe (DHT) dem halben gemessenen Wert. Ab einem gemessenen Wert von DHT/2 ≥ 3,5 mm darf ein Material als Bulk-Fill-fähig bezeichnet werden und eine Durchhärtungstiefe von mindestens 4 mm unter zahnärztlichen Bedingungen gilt als gesichert. Da durch die Einfärbung die Durchhärtungstiefe in der Regel verschlechtert wird, müssen Bulk-Fill-fähige Materialien in der uneingefärbten Form (Clearpaste) mindestens eine Durchhärttiefe DHT/2 von 4,5 mm oder mehr zu erreichen.

Die Vickershärte wurde mit einem Vickershärtemessgerät der Firma Zwick (ZHV 0.2) bestimmt. Zusätzlich wird der Durchhärtungstiefe [in mm] angegeben, bei der die Vickershärte eines polymerisierten, quer bis zur Mitte abgeschliffenen Prüfkörpers noch 80 % der Oberflächenhärte beträgt.

Die Röntgenopazität, der CR-Wert, die Glanzstabilität, die Polierbarkeit und die Oberflächenrauheit wurden auf die in der Beschreibung beschriebene Weise bestimmt.
- Beschleuniger: Ethyl-4-(dimethylamino)benzoat (CAS-Nr. 10287-53-3)
- Bis-GMA: Bisphenol-A-glycidylmethacrylat (CAS-Nr. 1565-94-2)
- TCP: Tricyclodecandimethanoldiacrylat (CAS-Nr. 42594-17-2)
- MA836: 2-([1,1'-Biphenyl]-2-oxy)ethylmethacrylat
- Ge-Photoinitiator: Bis(4-methoxybenzoyl)diethylgermanium (CAS-Nr.1469766-31-1)
- Glasfüller: Barium-freies Sr-, Al- und F-haltiges Dentalglas mit 6 % Silanisierung, mittlere Korngröße 0,7 µm, Brechungsindex 1,50 (Glas G018-163)
- Kettelregler: 2-(Toluol-4-sulfonylmethyl)acrylsäureethylester (MA-769)
- RM3: 7,7(9)9-Trimethyl-4,3-dioxo-3,14-dioxa-5,12-diazohexadecan-1,16-diyldimethacrylat

- SiO₂/ZrO₂-Mischoxid: sphärische SiO₂/ZrO₂-Mischoxidpartikel, mittlere Primärpartikelgröße: 3 bis 30 nm, Sekundärpartikelgröße: 3 bis 15 µm, Brechungsindex 1,50
- SR-348C: ethoxyliertes Bisphenol-A-dimethacrylat (CAS-Nr 41637-38-1)
- V380: Urethandimethacrylat mit aromatischen Gruppen
- nYbF₃: nanoskaliges Ytterbiumtrifluorid, mittlere Partikelgröße 10-24 nm (zahlengemittelter D50-Wert)
- V837: N-(2-Methacryloyloxyethyl)carbaminsäure-(2-methacryloyloxyethyl)-ester (CAS-Nr. 139096-43-8)
- CC: Campherchinon

### Beispiel 1

### Herstellung eines röntgenopaken Kompositfüllers mit sphärischen Partikeln

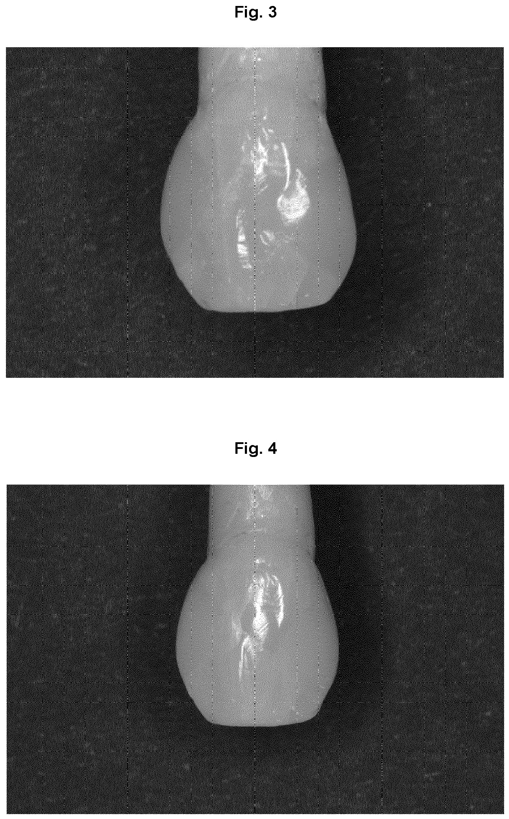

Zur Herstellung eines Kompositfüllers mit der in Tabelle 1 angegebenen Zusammensetzung wurden zunächst die in der Tabelle genannten Monomere miteinander gemischt und dann das SiO₂/ZrO₂-Mischoxid in die Monomermischung eingearbeitet. Das Dispergieren erfolgte in einem zylindrischen Glas durch moderates Rühren für 6 bis 24 Stunden. Im Anschluss wurden Campherchinon und Ethyl-4-(dimethylamino)benzoat (Beschleuniger) zugefügt und weiter gerührt, bis sich die Initiatorkomponenten gelöst hatten. Die Mischung wurde dann mit 20 ml/min in eine Zerstäuberdüse gepumpt, welche mit einem Druck von 2,1 bar unter Stickstoff betrieben wurde. Die fein zerstäubten Tröpfchen wurden mit sechs 100 Watt-LED-Lampen der Wellenlänge 470 nm polymerisiert. Die Größe der gehärteten Partikel wurde mittels Laserbeugung bestimmt (Microtrac X-100 Partikel-Messgerät). Die Partikel hatten eine sphärische Struktur und eine durchschnittliche Partikelgröße von 20 µm. Die Partikelgröße kann durch die Zugabe von Aceton zur Monomermischung vor dem Versprühen (0 bis 25 %) kontrolliert werden. Figur 4 zeigt eine rasterelektronenmikroskopische Aufnahme der sphärischen Partikel. Der Kompositfüller hat einen Brechungsindex von 1,502.

**Tabelle 1: Zusammensetzung des röntgenopaken sphärischen Kompositfüllers**

| **Komponente** | **Anteil [Gew.-%]** |
|---|---|
| V380 | 7,37 |
| RM3 | 7,83 |
| D3MA | 19,16 |
| nYbF3 | 9,85 |
| CC und Beschleuniger | 0,41 |
| SiO₂/ZrO₂-Mischoxid | 55,00 |
| Additive | 0,39 |
| Summe | 100 |

### Beispiel 2

### Herstellung eines erfindungsgemäßen Dentalwerkstoffs

Zur Herstellung eines Dentalwerkstoffs mit der in Tabelle 2 angegebenen Zusammensetzung wurden zunächst die genannten Monomere unter Rühren homogen vermischt und im Anschluss das Ytterbiumtrifluorid in einen Teil der Mischung eingearbeitet, so dass eine weitgehend transparente Flüssigkeit erhalten wurde. Im Anschluss wurden die restlichen Monomere und danach die pulverförmigen Komponenten zugegeben und homogen zu einer Paste vermischt. Der Werkstoff wurde auf die oben beschriebene Weise analysiert. Die Ergebnisse sind in Tabelle 3 angegeben.

**Tabelle 2: Zusammensetzung der röntgenopaken Kompositpaste**

| **Komponente** | **Anteil [Gew.-%]** |
|---|---|
| Kompositfüller (Beispiel 1) | 24,85 |
| SiO2/ZrO2-Mischoxid | 9,94 |
| Glasfüller | 38,07 |
| V380 | 4,14 |
| SR-348C | 2,83 |
| TCP | 5,51 |
| V837 | 0,56 |
| MA-836 | 1,44 |
| RM3 | 3,65 |
| Kettenregler | 0,99 |
| CC | 0,04 |
| Beschleuniger | 0,15 |
| Ge-Photoinitiator | 0,03 |
| Additive | 0,64 |
| nYbF3 | 7,17 |
| Summe | 100 |

**Tabelle 3: Eigenschaften des gehärteten Dentalwerkstoffs**

| **Messgröße** | **Werkstoff** |
|---|---|
| DHT/2 (mm) | 5,0 |
| Transmission (%) | 13,5 |
| Biegefestigkeit (MPa) | 114 |
| Durchhärtungstiefe bei 80% Vickers-Härte [mm] | 6,2 |
| CR-Wert | 61,7 |
| Röntgenopazität (%Al) | 205 |
| Glanzstabilität (GE) | 44 |
| Polierbarkeit (GE) | 74 |
| Oberflächenrauheit (µm) | 0,11 |

### Beispiel 3

### Herstellung eines erfindunasaemäßen Dentalwerkstoffs

Zur Herstellung eines Dentalwerkstoffs mit der in Tabelle 4 angegebenen Zusammensetzung wurden zunächst die genannten Monomere unter Rühren homogen vermischt und im Anschluss das Ytterbiumtrifluorid in einen Teil der Mischung eingearbeitet, so dass eine weitgehend transparente Flüssigkeit erhalten wurde. Im Anschluss wurden die restlichen Monomere und danach die pulverförmigen Komponenten zugegeben und homogen zu einer Paste vermischt. Der Werkstoff wurde auf die oben beschriebene Weise analysiert. Die Ergebnisse sind in Tabelle 5 angegeben.

**Tabelle 4: Zusammensetzung der röntgenopaken Kompositpaste**

| **Komponente** | **Anteil [Gew.-%]** |
|---|---|
| Kompositfüller | 19,73 |
| SiO2/ZrO2-Mischoxid | 9,87 |
| Glasfüller | 42,72 |
| V380 | 4,21 |
| SR-348C | 2,88 |
| TCP | 5,63 |
| V837 | 0,57 |
| MA-836 | 1,47 |
| RM3 | 3,71 |
| Kettenregler | 1,01 |
| CC | 0,04 |
| Beschleuniger | 0,15 |
| Ge-Photoinitiator | 0,04 |
| Additive | 0,64 |
| nYbF3 | 7,33 |
| Summe | 100 |

**Tabelle 5: Eigenschaften des gehärteten Dentalwerkstoffs**

| **Messgröße** | **Werkstoff** |
|---|---|
| DHT/2 (mm) | 5,0 |
| Transmission (%) | 13,1 |
| Biegefestigkeit (MPa) | 124 |
| Durchhärtungstiefe bei 80% Vickers-Härte [mm] | 6,0 |
| CR-Wert | 62,5 |
| Röntgenopazität (%Al) | 202 |
| Glanzstabilität (GE) | 50 |
| Polierbarkeit (GE) | 76 |
| Oberflächenrauheit (µm) | 0,10 |

### Beispiel 4

### Herstellung eines erfindunasaemäßen Dentalwerkstoffs

Zur Herstellung eines Dentalwerkstoffs mit der in Tabelle 6Tabelle angegebenen Zusammensetzung wurden zunächst die genannten Monomere unter Rühren homogen vermischt und im Anschluss das Ytterbiumtrifluorid in einen Teil der Mischung eingearbeitet, so dass eine weitgehend transparente Flüssigkeit erhalten wurde. Im Anschluss wurden die restlichen Monomere und danach die pulverförmigen Komponenten zugegeben und homogen zu einer Paste vermischt. Der Werkstoff wurde auf die oben beschriebene Weise analysiert. Die Ergebnisse sind in Tabelle 7 angegeben.

**Tabelle 6: Zusammensetzung der röntgenopaken Kompositpaste**

| **Komponente** | **Anteil [Gew.-%]** |
|---|---|
| Kompositfüller | 30,00 |
| SiO₂/ZrO₂-Mischoxid | 10,00 |
| Glasfüller | 33,30 |
| V380 | 4,08 |
| SR-348C | 2,80 |
| TCP | 5,41 |
| V837 | 0,55 |
| MA-836 | 1,42 |
| RM3 | 3,60 |
| Kettenregler | 0,97 |
| CC | 0,04 |
| Beschleuniger | 0,15 |
| Ge-Photoinitiator | 0,03 |
| Additive | 0,63 |
| nYbF3 | 7,03 |
| Summe | 100 |

**Tabelle 7: Eigenschaften des gehärteten Dentalwerkstoffs**

| **Messgröße** | **Werkstoff** |
|---|---|
| DHT/2 (mm) | 4,8 |
| Transmission (%) | 13,6 |
| Biegefestigkeit (MPa) | 110 |
| Durchhärtungstiefe bei 80 % Vickers-Härte [mm] | 6,2 |
| CR-Wert | 60,9 |
| Röntgenopazität (%Al) | 182 |
| Glanzstabilität (GE) | 54 |
| Polierbarkeit (GE) | 75 |
| Oberflächenrauheit (µm) | 0,105 |

### Einfärbung der Paste in eine für dunkle Zähne geeignete Farbe

Die Kompositpaste wurde durch schrittweise Zugabe der Pigmente Sicotransrot, Sicotransgelb, Xerogel gelb und Sicovitschwarz auf die folgenden L,a,b,CR-Werte eingestellt. Anschließend wurden die Transmission und Durchhärtungstiefe gemessen.

| L* | a* | b* | CR | Transmission | DHT/2 |
|---|---|---|---|---|---|
| 79,7 | 9,6 | 33,7 | 63,5 | 13,3 % | 3,8mm |

Mit einer Durchhärtungstiefe von 3,8 mm ist das Material gemäß ISO4049:2019 ein Bulk-Fill Material, das gemäß Figur 3 eine vergleichbare Ästhetik erreicht wie ein konventionelles ästhetisches 2 mm Material, aber den Vorteil hat, dass es in Schichtdicke von 4 mm ausgehärtet werden kann und somit ein hocheffizientes Arbeiten im Seitenzahn ermöglicht.

### Beispiel 5

### Herstellung eines erfindungsgemäßen Dentalwerkstoffs

Zur Herstellung eines Dentalwerkstoffs mit der in Tabelle 8 angegebenen Zusammensetzung wurden zunächst die genannten Monomere unter Rühren homogen vermischt und im Anschluss das Ytterbiumtrifluorid in einen Teil der Mischung eingearbeitet, so dass eine weitgehend transparente Flüssigkeit erhalten wurde. Im Anschluss wurden die restlichen Monomere und danach die pulverförmigen Komponenten zugegeben und homogen zu einer Paste vermischt. Der Werkstoff wurde auf die oben beschriebene Weise analysiert. Die Ergebnisse sind in Tabelle 9 angegeben.

**Tabelle 8: Zusammensetzung der röntgenopaken Kompositpaste**

| **Komponente** | **Anteil [Gew.-%]** |
|---|---|
| Kompositfüller | 19,81 |
| SiO₂/ZrO₂-Mischoxid | 9,89 |
| Glasfüller | 42,58 |
| V380 | 4,36 |
| SR-348C | 2,97 |
| TCP | 5,85 |
| V837 | 0,59 |
| MA-836 | 1,53 |
| RM3 | 3,83 |
| Kettenregler | 0,73 |
| CC | 0,04 |
| Beschleuniger | 0,16 |
| Ge-Photoinitiator | 0,04 |
| Additive | 0,63 |
| nYbF3 | 6,99 |
| Summe | 100 |

**Tabelle 9: Eigenschaften des gehärteten Dentalwerkstoffs**

| **Messgröße** | **Werkstoff** |
|---|---|
| DHT/2 (mm) | 4,6 |
| Transmission (%) | 13,6 |
| Biegefestigkeit (MPa) | 117 |
| Durchhärtungstiefe bei 80 % Vickers-Härte [mm] | 7,0 |
| CR-Wert | 62,8 |
| Röntgenopazität (%Al) | 202 |
| Glanzstabilität (GE) | 49 |
| Polierbarkeit (GE) | 72 |
| Oberflächenrauheit (µm) | 0.12 |

Tabelle 9 ist zu entnehmen, dass Beispiel 5 einen hervorragenden Kompromiss aus Ästhetik (geringe Transmission von weniger als 14, hohe CR-Wert über 62, gute Glanzstabilität von 49, sehr gute Polierbarkeit von über 70 und geringe Oberflächenrauheit) und hoher Durchhärtungstiefe der Clearpaste (4, 6 mm gemäß ISO4049; noch in 7,0 mm Tiefe eine Vickershärte von 80% bezogen auf die Oberflächenhärte) darstellt.

### Beispiel 6

### Herstellung eines erfindunasaemäßen Dentalwerkstoffs

Zur Herstellung eines Dentalwerkstoffs mit der in Tabelle 10 angegebenen Zusammensetzung wurden zunächst die genannten Monomere unter Rühren homogen vermischt und im Anschluss das Ytterbiumtrifluorid in einen Teil der Mischung eingearbeitet, so dass eine weitgehend transparente Flüssigkeit erhalten wurde. Im Anschluss wurden die restlichen Monomere und danach die pulverförmigen Komponenten zugegeben und homogen zu einer Paste vermischt. Der Werkstoff wurde auf die oben beschriebene Weise analysiert. Die Ergebnisse sind in Tabelle 11 angegeben.

**Tabelle 10: Zusammensetzung der röntgenopaken Kompositpaste**

| **Komponente** | **Anteil [Gew.-%]** |
|---|---|
| Kompositfüller | 20,13 |
| SiO₂/ZrO₂-Mischoxid | 10,07 |
| Glasfüller | 41,61 |
| V380 | 4,43 |
| SR-348C | 3,02 |
| TCP | 6,20 |
| MA-836 | 1,56 |
| RM3 | 4,24 |
| Kettenregler | 0,75 |
| CC | 0,04 |
| Beschleuniger | 0,16 |
| Ge-Photoinitiator | 0,04 |
| Additive | 0,64 |
| nYbF3 | 7,11 |
| Summe | 100 |

**Tabelle 11: Eigenschaften des gehärteten Dentalwerkstoffs**

| **Messgröße** | **Werkstoff** |
|---|---|
| DHT/2 (mm) | 4,5 |
| Transmission (%) | 14,0 |
| Biegefestigkeit (MPa) | 119 |
| Durchhärtungstiefe bei 80 % Vickers-Härte [mm] | 6,3 |
| CR-Wert | 62,0 |
| Röntgenopazität (%Al) | 201 |
| Glanzstabilität (GE) | 44 |
| Polierbarkeit (GE) | 76 |
| Oberflächenrauheit (µm) | 0.10 |

### Einfärbung der Paste in eine für dunkle Zähne geeignete Farbe

Die Kompositpaste aus Beispiel 6 wurde durch schrittweise Zugabe der Pigmente Sicotransrot, Sicotransgelb, Xerogel gelb, Echtrot und Sicovitschwarz auf die folgenden L,a,b,CR-Werte eingestellt. Anschließend wurden die Transmission und Durchhärtungstiefe gemessen.

| L* | a* | b* | CR | Transmission | DHT/2 |
|---|---|---|---|---|---|
| 77,4 | 12,0 | 32,8 | 66,9 | 12,8 % | 3,6 mm |

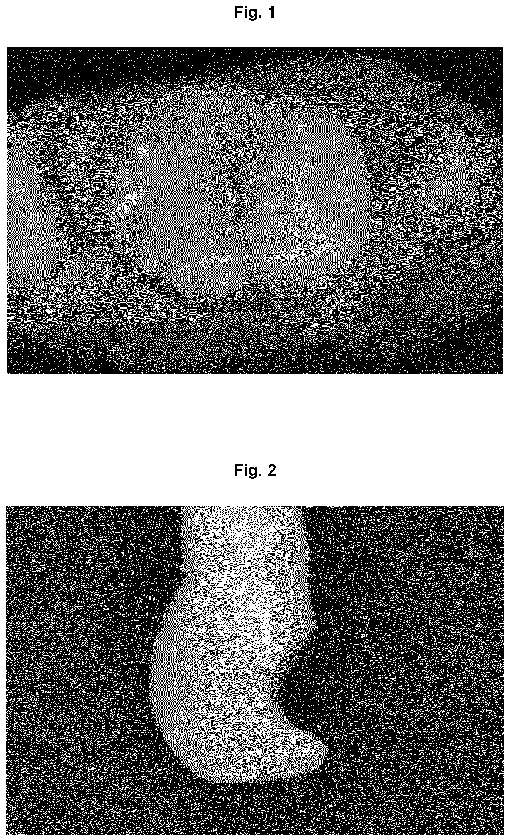

Mit einer Durchhärtungstiefe von 3,6 mm und einer Härte von 80% (bezogen auf den Wert in 0,5 mm Tiefe) in einer Tiefe von 5,2 mm ist das Material gemäß ISO4049:2019 ein Bulk-Fill Material, das gemäß Fig. 2 und Fig.3 eine vergleichbare Ästhetik erreicht wie ein konventionelles ästhetisches 2 mm Material.

### Beispiel 7

### Herstellung eines erfindungsgemäßen Dentalwerkstoffs

Zur Herstellung eines Dentalwerkstoffs mit der in Tabelle 12 angegebenen Zusammensetzung wurden zunächst die genannten Monomere unter Rühren homogen vermischt und im Anschluss das Ytterbiumtrifluorid in einen Teil der Mischung eingearbeitet, so dass eine weitgehend transparente Flüssigkeit erhalten wurde. Im Anschluss wurden die restlichen Monomere und danach die pulverförmigen Komponenten zugegeben und homogen zu einer Paste vermischt. Der Werkstoff wurde auf die oben beschriebene Weise analysiert. Die Ergebnisse sind in Tabelle 13 angegeben.

**Tabelle 12: Zusammensetzung der röntgenopaken Kompositpaste**

| **Komponente** | **Anteil [Gew.-%]** |
|---|---|
| Kompositfüller | 20,00 |
| SiO₂/ZrO₂-Mischoxid | 10,00 |
| Glasfüller | 41,90 |
| V380 | 2,62 |
| SR-348C | 4,30 |
| TCP | 5,93 |
| V837 | 0,60 |
| RM3 | 4,14 |
| Kettenregler | 0,75 |
| CC | 0,04 |
| Beschleuniger | 0,16 |
| Ge-Photoinitiator | 0,04 |
| Additive | 0,64 |
| nYbF3 | 7,09 |
| Bis-GMA | 1,80 |
| Summe | 100 |

**Tabelle 13: Eigenschaften des gehärteten Dentalwerkstoffs**

| **Messgröße** | **Werkstoff** |
|---|---|
| DHT/2 (mm) | 4,6 |
| Transmission (%) | 13,7 |
| Biegefestigkeit (MPa) | 124 |
| CR-Wert | 62,3 |

### Beispiel 8

### Herstellung eines erfindunasaemäßen Dentalwerkstoffs

Zur Herstellung eines Dentalwerkstoffs mit der in Tabelle 14 angegebenen Zusammensetzung wurden zunächst die genannten Monomere unter Rühren homogen vermischt und im Anschluss das Ytterbiumtrifluorid in einen Teil der Mischung eingearbeitet, so dass eine weitgehend transparente Flüssigkeit erhalten wurde. Im Anschluss wurden die restlichen Monomere und danach die pulverförmigen Komponenten zugegeben und homogen zu einer Paste vermischt. Der Werkstoff wurde auf die oben beschriebene Weise analysiert. Die Ergebnisse sind in Tabelle 15 angegeben.

**Tabelle 14: Zusammensetzung der röntgenopaken Kompositpaste**

| **Komponente** | **Anteil [Gew.-%]** |
|---|---|
| Kompositfüller | 20,00 |
| SiO₂/ZrO₂-Mischoxid | 10,00 |
| Glasfüller | 41,90 |
| V380 | 2,62 |
| SR-348C | 2,08 |
| TCP | 5,40 |
| V837 | 0,60 |
| MA-836 | 1,43 |
| RM3 | 5,46 |
| Kettenregler | 0,75 |
| CC | 0,04 |
| Beschleuniger | 0,16 |
| Ge-Photoinitiator | 0,04 |
| Additive | 0,64 |
| nYbF3 | 7,09 |
| Bis-GMA | 1,80 |
| Summe | 100 |

**Tabelle 15: Eigenschaften des gehärteten Dentalwerkstoffs**

| **Messgröße** | **Werkstoff** |
|---|---|
| DHT/2 (mm) | 5,0 |
| Transmission (%) | 14,1 |
| Biegefestigkeit (MPa) | 108 |
| CR-Wert | 61,1 |

## Patentansprüche

1. Dentalwerkstoff, der
(a) mindestens ein radikalisch polymerisierbares Monomer,
(b) mindestens einen röntgenopaken Füllstoff,
(c) mindestens einen anorganischen Füllstoff,
(d) mindestens einen Kompositfüllstoff und
(e) mindestens einen Initiator für die radikalische Polymerisation enthält,
**dadurch gekennzeichnet, dass er** als Monomer (a)
(a-1) mindestens ein Urethan(meth)acrylat,
(a-2) mindestens ein radikalisch polymerisierbaren Bisphenol A-Derivat,
(a-3) ggf. mindestens ein polycyclisch-aliphatisches Dimethacrylat,
(a-4) ggf. mindestens ein Monomer, das nicht in eine der Gruppen (a-1) bis (a-3) und (a-5) fällt, und
(a-5) ggf. mindestens einen Kettenregler enthält.

2. Dentalwerkstoff nach Anspruch 1, der
(a-1) 20 bis 80 Gew.-%, bevorzugt 25 bis 55 Gew.-% und ganz besonders bevorzugt 30 bis 50 Gew.-% mindestens eines Urethan(meth)acrylats,
(a-2) 8 bis 45 Gew.-%, besonders bevorzugt von 10 bis 35 Gew.-% und ganz besonders bevorzugt 12 bis 35 Gew.-% mindestens eines radikalisch polymerisierbaren Bisphenol A-Derivats,
(a-3) 10 bis 50 Gew.-%, besonders bevorzugt von 15 bis 40 Gew.-% und ganz besonders bevorzugt 20 bis 35 Gew.-%, mindestens eines polycyclisch-aliphatischen, vorzugsweise tricyclisch-aliphatischen Dimethacrylats,
(a-4) 0 bis zu 20 Gew.-%, besonders bevorzugt 2 bis 20 Gew.-% und ganz besonders bevorzugt von 4 bis 10 Gew.-% sonstige Monomere, d.h. Monomere, die nicht in eine der Gruppen (a-1) bis (a-3) und (a-5) fallen, und
(a-5) 0 bis zu 8 Gew.-%, vorzugsweise 0,5 bis 7 Gew.-% und besonders bevorzugt 1 bis 6 Gew.-% mindestens eines Kettenreglers, enthält.
jeweils bezogen auf die Gesamtmasse der Komponente (a).

3. Dentalwerkstoff nach Anspruch 1 oder 2, der als **Urethan(meth)acrylat (a-1)**
- ein oder mehrere Urethandimethacrylatmonomere mit aromatischen Gruppen, vorzugsweise ein Urethandi(meth)acrylatderivat von 1,3-Bis(1-isocyoanato-1-methylethyl)benzol, besonders bevorzugt Tetramethyl-xylylen-diurethandi(meth)acrylat (V380),
- ein oder mehrere Urethandi(meth)acrylate der allgemeinen Formel I: 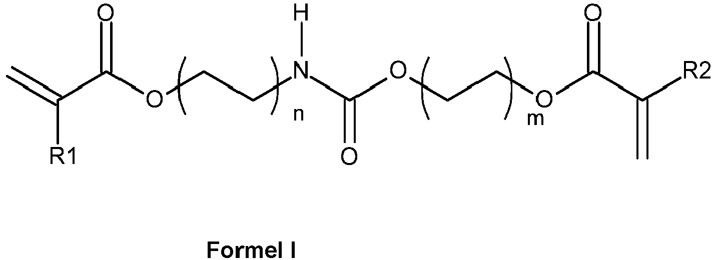 in der die Variablen die folgenden Bedeutungen haben:
R¹, R² = unabhängig voneinander jeweils C₁-C₈-Alkyl, vorzugsweise C₁-C₄-Alkyl, besonders bevorzugt C₁-C₂-Alkyl, ganz besonders bevorzugt CH₃;
n, m = unabhängig voneinander jeweils eine ganze Zahl von 1 bis 2 und bevorzugt 1,
- ein oder mehrere weitere Urethandi(meth)acrylate, vorzugsweise ein Urethandimethacrylat, besonders bevorzugtes Urethandimethacrylat ist 7,7(9)9-Trimethyl-4,3-dioxo-3,14-dioxa-5,12-diazohexadecan-1,16-diyl-dimethacrylat (RM3),
oder eine beliebige Mischung davon enthält.

4. Dentalwerkstoff nach Anspruch 2 oder 3, der als **Urethan(meth)acrylat (a-1)**
- 5 bis 60 Gew.-%, bevorzugt von 10 bis 45 Gew.-% und besonders bevorzugt 10 bis 25 Gew.-% mindestens eines Urethandimethacrylatmonomers mit aromatischen Gruppen, vorzugsweise V380,
- 0 bis zu 5 Gew.-%, bevorzugt 0,01 bis zu 5 Gew.-% und besonders bevorzugt 0,04 bis 4 Gew.-% mindestens eines Urethandi(meth)acrylats der Formel I, vorzugsweise V837 und
- 10 bis 70 Gew.-%, besonders bevorzugt 15 bis 45 Gew.-% und ganz besonders bevorzugt 15 bis 35 Gew.-% mindestens eines weiteren Urethandimethacrylats, vorzugsweise RM3, enthält,
jeweils bezogen auf die Gesamtmasse der Monomerkomponente (a).

5. Dentalwerkstoff nach einem der Ansprüche 1 bis 4, der
als **radikalisch polymerisierbares Bisphenol A-Derivat (a-2)** 2,2-Bis[4-(2-hydroxy-3-methacryloyloxypropyl)phenyl]propan (Bis-GMA), bevorzugt Bisphenol-A-dimethacrylat, besonders bevorzugt ethoxyliertes oder propoxyliertes Bisphenol-A-Dimethacrylat und ganz besonders bevorzugt 2-[4-(2-Methacryloyloxyethoxyethoxy)phenyl]-2-[4-(2-methacryloyloxyethoxy)phenyl]propan) (SR-348c) oder eine Mischung davon, und/oder
als **polycyclisch-aliphatisches Dimethacrylat (a-3)** ein Tricyclodecandimethanoldimethacrylat, bevorzugt Tricyclodecandimethanoldimethacrylat TCP (CAS-Nummer: 42594-17-2), und/oder
als **weiteres Monomer (a-4)** mindestens ein (Meth)acrylamid, vorzugsweise ein N-disubstituiertes (Meth)acrylamid, wie z.B. N,N-Dimethylacrylamid, mindestens ein Bis(meth)acrylamid, wie N,N'-Diethyl-1,3-bis(acrylamido)propan, 1,3-Bis(methacryl-amido)-propan, 1,4-Bis(acrylamido)-butan oder 1,4-Bis(acryloyl)piperazin, besonders bevorzugt mindestens ein polyfunktionelles, vorzugsweise difunktionelles, Methacrylat, wie Di-, Tri- oder Tetraethylenglycoldimethacrylat, Trimethylolpropantrimethacrylat, Pentaerythrittetramethacrylat, Glycerindimethacrylat und Glycerintrimethacrylat, 1,4-Butandioldimethacrylat, 1,10-Decandioldimethacrylat (D₃MA), 1,12-Dodecandioldimethacrylat und ganz besonders bevorzugt mindestens ein monofunktionelles Methacrylat, bevorzugt 2-([1,1'-Biphenyl]-2-oxy)ethylmethacrylat (MA836), oder eine Mischung davon enthält.

6. Dentalwerkstoff nach Anspruch 5, der
- 8 bis 45 Gew.-%, besonders bevorzugt von 10 bis 35 Gew.-% und ganz besonders bevorzugt 12 bis 35 Gew.-% mindestens eines Bisphenol A-Derivats (a-2), und/oder
- 10 bis 50 Gew.-%, besonders bevorzugt von 15 bis 40 Gew.-% und ganz besonders bevorzugt 20 bis 35 Gew.-% mindestens eines tricyclischen Dimethacrylats (a-3), und/oder
- maximal 20 Gew.-%, besonders bevorzugt 2 bis 20 Gew.-% und ganz besonders bevorzugt von 4 bis 10 Gew.-% mindestens eines Monomers (a-4) enthält,
jeweils bezogen auf die Masse der Monomerkomponente (a).

7. Dentalwerkstoff nach einem der Ansprüche 1 bis 6, der als **Kettenregler (a-5)** mindestens einen Arylsulfonylalkylacrylsäurealkylester, 2-(Toluol-4-sulfonylmethyl)-acrylsäureethylester (TSMEA), 2-(Toluol-4-sulfonylmethyl)-acrylsäure-2-(2-ethoxy-ethoxy)-ethylester, 2-(Toluol-4-sulfonylmethyl)-acrylsäure, 2-(Toluol-4-sulfonylmethyl)-acrylsäuretert-butylester, 3-(Trimethoxysilyl)propyl-2-(tosylmethyl)acrylat, 3-(Triethoxysilyl)propyl-2-(tosylmethyl)acrylat, 3-(Triethoxysilyl)propyl-2-(tosylmethyl)acrylamid, 2-(Methylsulfonyl)-ethylmethacrylat, 2-(Methylsulfonyl)-ethylmethacrylat, 3-(Methylsulfonyl)-propylmethacrylat, Triethylenglycol-bis[2-(toluol-4-sulfonylmethyl)-acrylat], 2-(Toluol-4-sulfonylmethyl)-acrylsäure-(2-methacryloyloxyethyl)ester, 2-(Toluol-4-sulfonylmethyl)-acrylsäure-(6-methacryloyloxyhexyl)ester, 2-(Toluol-4-sulfonylmethyl)acrylsäure-(10-methacryloyloxydecyl)ester, 2-(Toluol-4-sulfonylmethyl)-acrylsäure-(2-hydroxy-3-methacryloyloxypropyl)ester, 2-(Toluol-4-sulfonylmethyl)-acrylsäure-(8-methacryloyloxy-3,6-dioxaoctyl)ester enthält,
vorzugsweise in einer Menge von 0 bis 8 Gew.-%, besonders bevorzugt von 0,1 bis 7 Gew.-% und ganz besonders bevorzugt von 0,5 bis 6 Gew.-%, bezogen auf die Masse der Monomerkomponente (a).

8. Dentalwerkstoff nach einem der Ansprüche 1 bis 7, bei dem die Monomerkomponente (a) einen Brechungsindex von 1,495 bis 1,520, bevorzugt von 1,505 bis 1,515 hat, wobei der Brechungsindex der Monomermischung vorzugsweise so eingestellt ist, dass er vor der Härtung dem Brechungsindex des Füllstoffs (c) entspricht oder maximal 0,03 darüber liegt und vorzugsweise um 0,002 bis 0,02, besonders bevorzugt um 0,005 bis 0,015 größer als der Brechungsindex des Füllstoffs (c) ist.

9. Dentalwerkstoff nach einem der Ansprüche 1 bis 8, der als **Komponente (b)** Ytterbiumtrifluorid-Partikel mit einer zahlengemittelten Teilchengröße (D50) von ≤ 25 nm, vorzugsweise < 25 nm, besonders bevorzugt von 10 bis 24 nm, ganz besonders bevorzugt von 15 bis 24 nm enthält, und/oder
als **anorganischen Füllstoff (c)** mindestens ein Glaspulver, bevorzugt ein Strontiumglaspulver und/oder ein zirkonhaltiges Glaspulver enthält, vorzugsweise mit einer volumengemittelten Partikelgrößen (D50-Wert) von 0,1 bis 1,5 µm, besonders bevorzugt 0,3 bis 1,2 µm und ganz besonders bevorzugt 0,4 bis 0,9 µm, mindestens ein SiO₂/ZrO₂-Mischoxid, vorzugsweise mit einer volumengemittelten (D50-Wert) Primärteilchengröße von 2 bis 100 nm, bevorzugt 2 bis 60 nm, besonders bevorzugt 2 bis 40 nm und ganz besonders bevorzugt 3 bis 30 nm, und einer volumengemittelten (D50-Wert) Sekundärpartikelgröße 0,5 bis 30 µm, bevorzugt 2 bis 25 µm, besonders bevorzugt von 3 bis 20 µm und ganz besonders bevorzugt 3 bis 15 µm, oder eine Mischung davon enthält.

10. Dentalwerkstoff nach einem der Ansprüche 1 bis 9, der als Komponente (d) einen partikulären Kompositwerkstoff enthält, der durch Polymerisation der folgenden Zusammensetzung erhältlich ist:
- 8 bis 50 Gew.-%, vorzugsweise 18 bis 50 Gew.-% radikalisch polymerisierbares Monomer,
- 1 bis 20 Gew.-%, vorzugsweise 8 bis 13 Gew.-% Ytterbiumtrifluoridpartikel mit einer mittleren Teilchengröße von < 25 nm (zahlengemittelter D50-Wert),
- 40 bis 90 Gew.-%, vorzugsweise 50 bis 70 Gew.-% weitere anorganischer Füllstoffe, vorzugsweise SiO₂/ZrO₂-Mischoxid und
- 0,01 bis 2 Gew.-%, vorzugsweise 0,1 bis 1 Gew.-% Initiator für die radikalische Polymerisation,
jeweils bezogen auf die Gesamtmasse des Kompositfüllstoffs.

11. Dentalwerkstoff nach einem der Ansprüche 1 bis 10, der
- 5 bis 40 Gew.-%, vorzugsweise 10 bis 35 Gew.-%, besonders bevorzugt 12 bis 30 Gew.-% mindestens eines radikalisch polymerisierbaren Monomeren (a),
- 1 bis 30 Gew.-%, vorzugsweise 3 bis 10 Gew.-%, besonders bevorzugt 5 bis 10 Gew.-% Ytterbiumtrifluoridpartikel (b) mit einer mittleren Teilchengröße von < 25 nm (zahlengemittelter D50-Wert),
- 20 bis 90 Gew.-%, vorzugsweise 30 bis 70 Gew.-%, besonders bevorzugt 40 bis 65 Gew.-% anorganischen Füllstoff (c),
- 5 bis 60 Gew.-%, vorzugsweise 15 bis 50 Gew.-%, besonders bevorzugt 15 bis 40 Gew.-% Kompositfüllstoff (d) und
- 0,005 bis 3,0 Gew.-%, vorzugsweise 0,01 bis 2,0 Gew.-%, besonders bevorzugt 0,1 bis 1 Gew.-% Initiator für die radikalische Polymerisation (e) enthält,
jeweils bezogen auf die Gesamtmasse des Dentalwerkstoffs.

12. Dentalwerkstoffe nach einem der Ansprüche 1 bis 11, der die folgende Zusammensetzung aufweist:
(a) 5 bis 40 Gew.-% mindestens eines radikalisch polymerisierbaren Monomers,
(b) 1 bis 30 Gew.-% Ytterbiumtrifluorid mit einer mittleren Partikelgröße (zahlengemittelter D50-Wert) von < 25 nm als röntgenopaken Füllstoff
(c) 20 bis 90 Gew.-% mindestens eines anorganischen Füllstoffs,
(d) 5 bis 60 Gew.-% mindestens eines Kompositfüllstoffs und
(e) 0,01 bis 3,0 Gew.-% mindestens eines Initiators für die radikalische Polymerisation, vorzugsweise einen Photoinitiator, enthält,
jeweils bezogen auf die Masse des Dentalwerkstoffs,
wobei der Dentalwerkstoff als radikalisch polymerisierbares **Monomer (a)** eine Mischung von
(a-1) 20 bis 80 Gew.-% an Urethandimethacrylaten, die
- 5 bis 60 Gew.-% Tetramethylxylylendiurethandimethacrylat (V380),
- 5 bis 60 Gew-% sonstige Urethanmethacrylate, vorzugsweise RM3 enthalten,
(a-2) 5 bis 30 Gew.-% mindestens eines radikalisch polymerisierbaren Bisphenol-A-Dimethacrylats,
(a-3) 10 zu 50 Gew.-% mindestens eines polycyclisch-aliphatischen Dimethacrylats, vorzugsweise eines tricyclischen aliphatischen Dimethacrylats, ganz vorzugsweise Tricyclodecandimethanoldimethacrylat (TCP), und
(a-4) 0 bis zu 20 Gew.-% sonstiger Monomere, d.h. Monomere, die nicht in eine der Gruppen (a-1) bis (a-3) und (a-5) fallen, vorzugsweise mindestens eines monofunktionellen radikalisch polymerisierbaren Monomers, ganz bevorzugt 2-([1,1'-Biphenyl]-2-oxy)ethylmethacrylat (MA-836) enthält, und
(a-5) 0 bis zu 8 Gew.-% mindestens eines Kettenreglers, enthält,
jeweils bezogen auf die Gesamtmasse der Komponente (a), wobei die Monomerkomponente (a) einen Brechungsindex von 1,495 bis 1,520 hat,
als anorganischen **Füllstoff (c)**
- Glaspulver mit einem Brechungsindex im Bereich von 1,49 bis 1,52 und einer volumengemittelten Teilchengröße (D50-Wert) von 0,4 bis 0,9 µm, vorzugsweise von 0.4 bis 0.8 µm und ggf.
- ein oder mehrere SiO₂/ZrO₂-Mischoxide mit einem Brechungsindex in einem Bereich 1,490 von 1,510, vorzugsweise mit einer volumengemittelten (D50-Wert) Primärteilchengröße von 2 bis 100 nm, bevorzugt 2 bis 60 nm, besonders bevorzugt 2 bis 40 nm und ganz besonders bevorzugt 3 bis 30 nm, und einer volumengemittelten (D50-Wert) und einer Sekundärpartikelgröße von 0,5 bis 30 µm, bevorzugt 2 bis 25 µm, besonders bevorzugt von 3 bis 20 µm und ganz besonders bevorzugt 3 bis 15 µm, oder eine Mischung davon, und
als **Kompositfüllstoff (d)** einen Füllstoff mit einer volumengemittelten Teilchengröße (D50-Wert) von 5 bis 100 µm enthält, der durch Polymerisation einer Zusammensetzung hergestellt wird, die
- 8 bis 50 Gew.-%, vorzugsweise 18 bis 50 Gew.-% radikalisch polymerisierbares Monomer, das aus Glycerindimethacrylat, Alkylendimethacrylaten, wie 1,10-Decanedioldimethacrylat (D₃MA) und Triethylenglycoldimethacrylat (TEGDMA), Urethandimethacrylaten, wie 1,6-Bis-[2-methacryloyloxy-ethoxy-carbonylamino]-2,2,4-trimethylhexan (RM3), N-(2-Methacryloyloxyethyl)-carbaminsäure-(2-methacryloyloxyethyl)ester (V837) und Tetramethyl-xylylendiurethan-di(meth)acrylat (V380) und Mischungen davon ausgewählt ist,
- 8 bis 13 Gew.-% Ytterbiumtrifluoridpartikel mit einer zahlengemittelten Teilchengröße (D50-Wert) von < 25 nm,
- 40 bis 90 Gew.-% weiteren anorganischen Füllstoff und
- 0,01 bis 2 Gew.-% Initiator für die radikalische Polymerisation enthält,
jeweils bezogen auf die Gesamtmasse des Kompositfüllstoffs,
wobei die Partikelgröße in allen Fällen auf die in der Beschreibung beschriebene Weise gemessen wird, und
wobei der Brechungsindex der Monomerkomponente (a) dem Brechungsindex des Füllstoffs (c) entspricht oder maximal 0,03 größer ist und bei dem der Brechungsindex der Monomerkomponente (a) dem Brechungsindex des Kompositfüllstoffs (d) entspricht oder maximal 0,025 größer ist.

13. Dentalwerkstoff nach einem der vorigen Ansprüche, der
(a) 12 bis 30 Gew.-% mindestens eines radikalisch polymerisierbaren Monomers,
(b) 3 bis 10 Gew.-% Ytterbiumtrifluorid mit einer zahlengemittelten Partikelgröße (D50-Wert) von 10 bis 24 nm als röntgenopaken Füllstoff,
(c) 30 bis 70 Gew.-% mindestens eines anorganischen Füllstoffs,
(d) 15 bis 50 Gew.-% mindestens eines Kompositfüllstoffs und
(e) 0,01 bis 2,0 Gew.-% mindestens eines Initiators für die radikalische Polymerisation, vorzugsweise einen Photoinitiator, enthält,
jeweils bezogen auf die Masse des Dentalwerkstoffs,
wobei der Dentalwerkstoff als radikalisch polymerisierbares **Monomer (a)** eine Mischung von
(a-1) 20 bis 80 Gew.-% an Urethandimethacrylaten, die
- 5 bis 60 Gew.-% Tetramethylxylylendiurethandimethacrylat (V380), und
- 10 bis 70 Gew.-% 1,6-Bis-[2-methacryloyloxy-ethoxycarbonylamino]-2,2,4-trimethylhexan (RM3) enthalten,
(a-2) 8 bis 45 Gew.-% Bisphenol-A-Dimethacrylat 2-[4-(2-Methacryloyloxyethoxyethoxy)phenyl]-2-[4-(2-methacryloyloxyethoxy)phenyl]propan) (SR-348c), 2,2-Bis[4-(2-hydroxy-3-methacryloyloxypropyl)phenyl]propan (Bis-GMA), oder eine Mischung davon
(a-3) 15 bis 40 Gew.-% Bis-(3-methacryloyloxymethyl)tricyclo-[5.2.1.0^{2,6}]decan (TCP), und
(a-4) 4 bis 10 Gew.-% sonstiger Monomere, d.h. Monomere, die nicht in eine der Gruppen (a-1) bis (a-3) und (a-5) fallen, und
(a-5) 0,5 bis 7 Gew.-% mindestens eines Kettenreglers, enthält,
jeweils bezogen auf die Gesamtmasse der Komponente (a),
als anorganischen **Füllstoff (c)**
- bariumfreies Strontiumglaspulver mit einem Brechungsindex im Bereich von 1,49 bis 1,51 und einer volumengemittelten Teilchengröße (D50-Wert) von 0,4 bis 0,9 µm und/oder
- ein oder mehrere SiO₂/ZrO₂-Mischoxide mit einem Brechungsindex in einem Bereich 1,490 von 1,510, vorzugsweise mit einer volumengemittelten (D50-Wert) Primärteilchengröße von 2 bis 100 nm, bevorzugt 2 bis 60 nm, besonders bevorzugt 2 bis 40 nm und ganz besonders bevorzugt 3 bis 30 nm, und einer volumengemittelten (D50-Wert) und einer Sekundärpartikelgröße von 0,5 bis 30 µm, bevorzugt 2 bis 25 µm, besonders bevorzugt von 3 bis 20 µm und ganz besonders bevorzugt 3 bis 15 µm, oder eine Mischung davon und
als **Kompositfüllstoff (d)** einen Füllstoff mit einer volumengemittelten Teilchengröße (D50-Wert) von 5 bis 100 µm enthält, der durch Polymerisation einer Zusammensetzung hergestellt wird, die
- 18 bis 50 Gew.-% radikalisch polymerisierbares Monomer, das aus Glycerindimethacrylat, 1,10-Decanedioldimethacrylat (D₃MA), Triethylenglycoldimethacrylat (TEGDMA), 1,6-Bis-[2-methacryloyloxy-ethoxycarbonylamino]-2,2,4-trimethylhexan (RM3), N-(2-Methacryloyloxyethyl)carbaminsäure-(2-methacryloyloxyethyl)ester (V837),Tetramethyl-xylylendiurethan-di(meth)acrylat (V380) und Mischungen davon ausgewählt ist,
- 8 bis 13 Gew.-% Ytterbiumtrifluoridpartikel mit einer zahlengemittelten Teilchengröße (D50-Wert) von 10 bis 24 nm,
- 40 bis 90 Gew.-% Strontiumglaspulver und/oder SiO₂/ZrO₂-Mischoxid als weiteren anorganischen Füllstoff und
- 0,01 bis 2 Gew.-% Initiator für die radikalische Polymerisation enthält,
jeweils bezogen auf die Gesamtmasse des Kompositfüllstoffs.

14. Dentalwerkstoff nach einem der vorigen Ansprüche, der
- 15 bis 25 Gew.-% mindestens eines radikalisch polymerisierbaren Monomeren (a),
- 5 bis 10 Gew.-%Ytterbiumtrifluoridpartikel (b)
- 40 bis 55 Gew.-% anorganischen Füllstoff (c),
- 15 bis 40% Kompositfüllstoff (d) und
- 0,1 bis 0,5 Gew.-% Initiator für die radikalische Polymerisation (e) enthält,
jeweils bezogen auf die Masse des Dentalwerkstoffs.

15. Dentalwerkstoff nach einem der Ansprüche 1 bis 12, zur therapeutischen Anwendung als dentaler Zement, Beschichtungs- oder Verblendmaterial, vorzugsweise Füllungskomposit, besonders bevorzugt Bulk-Fill-Komposit.

16. Nicht therapeutische Verwendung eines Dentalwerkstoffs gemäß einem der Anspruch 1 bis 12 zur Herstellung von Inlays, Onlays, Kronen und Brücken.
